# EUROPEAN PATENT SPECIFICATION

(11) **EP 4 250 961 B1**
(45) Date of publication and mention of the grant of the patent: **04.11.2026**
(21) Application number: 21816095.0
(22) Date of filing: 30.11.2021
(51) Int. Cl.: A23P 10/30, A23P 10/40, A23K 20/174, A23K 30/20, A23L 33/155, A61P 3/02

(54) **NEW FORMULATIONS WITH REDUCED ANTIOXIDANT CONTENT, THEIR MANUFACTURE AND USE**
NEUE FORMULIERUNGEN MIT REDUZIERTEM ANTI-OXIDANS-GEHALT, IHRE HERSTELLUNG UND VERWENDUNG
NOUVELLES COMPOSITIONS À TENEUR RÉDUITE EN ANTI-OXIDANTS, LEUR FABRICATION ET UTILISATION

(30) Priority: 30.11.2020 EP 20210831
(43) Date of publication of application: 04.10.2023
(73) Proprietor: DSM IP Assets B.V., 6411 TE Heerlen (NL)
(72) Inventor: BUTZ, Jochen, Alexander, 4303 Kaiseraugst (CH); HERBRIG, Thomas, Joachim, 4303 Kaiseraugst (CH); PALAZOGLU KURK, Pelin, 4303 Kaiseraugst (CH); MERTIN, Frank, Theodor, 4303 Kaiseraugst (CH); POPP, Pascal, Urs, 4303 Kaiseraugst (CH); TSEKOU, Christos, 4303 Kaiseraugst (CH); URBAN, Kai, 4303 Kaiseraugst (CH); SCHAEFER, Christian, 4303 Kaiseraugst (CH)
(74) Representative: Kraus & Lederer PartGmbB
(86) International application number: PCT/EP2021/083505
(87) International publication number: WO 2022/112587

(56) References cited:
- EP-A1- 0 285 682
- EP-A2- 0 494 417
- WO-A1-2018/220340
- CN-A- 110 250 521
- US-A- 5 043 170
- US-A- 5 356 636
- US-A1- 2020 029 596
- US-B1- 6 540 085
- DHAKAL SHABANA PRAVEEN ET AL: "Microencapsulation of vitamins in food applications to prevent losses in processing and storage: A review", FOOD RESEARCH INTERNATIONAL, ELSEVIER, AMSTERDAM, NL, vol. 137, 23 May 2020 (2020-05-23), XP086360130, ISSN: 0963-9969, [retrieved on 20200523], DOI: 10.1016/J.FOODRES.2020.109326
- KATOUZIAN IMAN ET AL: "Nano-encapsulation as a promising approach for targeted delivery and controlled release of vitamins", TRENDS IN FOOD SCIENCE AND TECHNOLOGY, ELSEVIER SCIENCE PUBLISHERS, GB, vol. 53, 6 May 2016 (2016-05-06), pages 34 - 48, XP029579298, ISSN: 0924-2244, DOI: 10.1016/J.TIFS.2016.05.002
- GONNET M ET AL: "New trends in encapsulation of liposoluble vitamins", JOURNAL OF CONTROLLED RELEASE, ELSEVIER, AMSTERDAM, NL, vol. 146, no. 3, 15 September 2010 (2010-09-15), pages 276 - 290, XP027260358, ISSN: 0168-3659, [retrieved on 20100625]

## Description

### Summary of the invention

The present invention is directed to a formulation, especially a solid formulation, with a self-heating temperature ≥ 120°C comprising a high amount of gelatin, a low amount of antioxidant, Vitamin A or a derivative thereof and optionally Vitamin D or a derivative thereof, and, if present, low amounts of reducing sugars and polyhydric alcohols. The present invention is further directed to a process for the manufacture of such formulation, its use as additive to feed and premixes and feed additives, premixes and feed comprising such formulation.

When the formulation is prepared according to the process as disclosed below, it is also called "beadlets". It was a surprise that such formulation/beadlets with the advantageous properties as laid down below could be prepared with such low amounts of antioxidants, reducing sugars and polyhydric alcohols.

It was especially surprising that the self-heating temperature of such formulations/beadlets is increased though their antioxidant content is decreased compared to the formulations of the prior art. So far it was expected that an amount of at least 10 weight-% of an antioxidant such as e. g. BHT is needed to ensure a self-heating temperature of at least 120°C.

The invention is defined by the appended set of claims.

### Background of the invention

Formulations with oxidation sensitive compounds such as fat-soluble vitamins, especially vitamin A, vitamin D and any derivatives and mixtures thereof, have a potential for oxidation processes already at ambient conditions while storing or transporting such formulations. Depending on the reaction velocity of such exothermic degradation processes, the storage or transport temperature and the volume of the stored or transported formulation, heat accumulation can result which may lead to self-heating and even auto-ignition of such formulations.

To minimize the degradation processes and thus decreasing also the self-heating effect, the oxidation sensitive compounds have to be protected in a suitable matrix and form.

Formulations of vitamin A, vitamin D and any derivatives and mixtures thereof having a self-heating temperature ≥ 100°C, but lower than 120°C have to be stored and transported in flexible intermediate containers with a volume of < 450 l. Furthermore, they have to be classified as "Dangerous goods class 4.2" and special equipment has to be used for their storage and transport which adds to the overall costs of such formulation.

Formulations of vitamin A, vitamin D and any derivatives and mixtures thereof having a self-heating temperature ≥ 120°C (as measured as described below) are allowed to be stored and transported in **f**lexible **i**ntermediate **b**ulk **c**ontainer**s** ("FIBCs") of volumes up to 3000 l (= 3 m³), preferably in FIBCs of volumes ranging from 450 to 3000 l. Furthermore, no classification as "dangerous goods" is needed. For reasons of sustainability it is highly desired to use such FIBCs, i.e. such bigger bags of volumes up to 3000 l compared to smaller bags of volumes < 450 l : First, because due to their larger size, less bags are needed to store and transport the same amount of formulation. Second, since the FIBCs until now are not re-usable, less bags have to be discarded.

EP 0 285 682 A1 relates to a process for the manufacture of preparations containing fat-soluble vitamins and/or carotenoids, as well as with the preparations so obtained.

US 5 043 170 A relates to an animal feed composition containing Vitamin D3 metabolites in combination with other essential micronutrients like Vitamins D3 and A.

CN 110 250 521 A relates to a preparation method of vitamin A acetate microcapsules.

Thus, there is the need to provide a formulation of vitamin A, vitamin A derivatives and any mixtures thereof and any of their mixtures with vitamin D which has a self-heating temperature ≥ 120°C so that it can be stored and transported in containers, especially in FIBCs, of volumes up to 3000 l, preferably in FIBCs of volumes ranging from 450 to 3000 l, more preferably in FIBCs of volumes ranging from 480 to 2000 l, even more preferably in FIBCs of volumes ranging from 500 to 1500 l and from 500 to 1000 l, most preferably in FIBCs of volumes ranging from 600 to 800 l.

### Detailed description

Thus, this need is fulfilled by the present invention, which is directed to a formulation with a self-heating temperature ≥ 120°C comprising
a) a fat-soluble vitamin in an amount of at least 25 weight-%, whereby the fat-soluble vitamin is vitamin A or a derivative thereof and optionally vitamin D or a derivative thereof;
b) gelatin in an amount of at least 45 weight-%;
c) at least one antioxidant in an amount of ≤ 7 weight-%;
d) an anti-caking agent;
e) optionally an oil;
f) optionally a reducing sugar;
g) optionally a polyhydric alcohol;
h) optionally residual moisture;
whereby all amounts a) to g) sum up to 100 weight-% and are based on the total weight of the amounts of a), b), c), d), e), f) and g) together.

The amount of residual moisture is preferably at most 6 weight-%, more preferably at most 5 weight-%, even more preferably at most 3.5 weight-%, most preferably at most 2 weight-%, based on the total weight of the formulation, i.e. the total weight of a) to h). The minimum amount of moisture in the formulation is preferably 0.1 weight-%, based on the total weight of the formulation.

When step E) of the process according to the present invention is performed the moisture content is lower than without step E) being performed.

Preferably, the formulations of the present invention do not comprise a lignosulfonate.

Though the amount of antioxidant is low in these formulations, they show a self-heating temperature ≥ 120°C. Furthermore, they show a good stability and a good homogeneity in premixes, as well as a high stability under feed pelleting conditions. Since the formulations according to the present invention show a self-heating temperature ≥ 120°C and a ΔT < 60 K, they can be transported in containers, especially in FIBCs ("**f**lexible **i**ntermediate **b**ulk **c**ontainer"), of volumes up to 3000 l, preferably in containers, especially in FIBCs, of volumes ranging from 450 l to 3000 l.

### Determination of the self-heating temperature

The tests are performed according to Norm E-15188 and VDI 2263 Part 1.

The samples are placed in a cubic shaped wire basket (either 16 ml or 1000 ml) in the center of an oven. The temperature of the oven is measured at two spots and remains constant during the testing period (24 hours), i.e. so called "isoperibolic test".

The temperature probe for the sample is placed in the center of the sample. Then the sample temperature reaches a temperature being 2 K below the oven temperature the 24 hour testing period starts.

When the temperature difference "ΔT" between sample and oven is < 60 K, the test criteria according to UN Orange book "Manual of Tests and Criteria, 6th revised edition, chapter 33.3, Division 4.2 is passed, and the sample is considered as being self-heating stable according to the present invention.

When the temperature difference "ΔT" between sample and oven is > 60 K, the sample is considered as undergoing spontaneous ignition or dangerous self-heating.

As illustrated by the examples, the formulations of the present invention show a self-heating temperature ≥ 120°C and a ΔT < 60 K, especially on large scale production of several thousand tons.

### Container

Any kind of container with a volume up to 3000 l, preferably with a volume ranging from 450 to 3000 l, more preferably with a volume ranging from 480 to 2000 l, even more preferably with a volume ranging from 500 to 1500 l and from 500 to 1000 l, most preferably with a volume ranging from 600 to 800 l, may be used.

A "**f**lexible intermediate **b**ulk **c**ontainer" ("FIBC"), "jumbo", "bulk bag", "super sack", "tote bag", or "big bag", is an industrial container made of flexible fabric that is designed for storing and transporting dry, flowable products, such as sand, fertilizer, and feed additives.

FIBCs are most often made of thick woven polyethylene or polypropylene, either coated or uncoated, and normally measure around 45-48 inches (114-122 cm) in diameter and varies in height from 100 to 200 cm (39 to 79 inches). Its capacity is normally around 1,000 kg or 2,200 lb, but the larger units can store even more. A bulk bag designed to transport one metric ton (0.98 long tons; 1.1 short tons) of material will itself only weigh 5-7 lb (2.3-5.0 kg).

Transporting and loading is done on either pallets or by lifting it from the loops. Bags are made with either one, two or four lifting loops. The single loop bag is suitable for one man operation as there is no need for a second man to put the loops on the loader hook. Emptying is made easy by a special opening in the bottom such as a discharge spout, of which there are several options, or by simply cutting it open.

Preferably a big bag with four lifting loops is used. Its volume preferably ranges from 480 to 2000 l, more preferably from 500 to 1500 l, even more preferably from 500 to 1000 l, most preferably from 600 to 800 l. A loading with 200 to 1500 kg of the formulation according to the present invention is preferred. More preferred is a loading with 300 to 1200 kg of the formulation according to the present invention. Most preferred is a loading with 500 to 900 kg of the formulation according to the present invention.

Thus, the present disclosure is also directed to a big bag, especially to a big bag with 3 or more lifting loops such as e. g. 4 lifting loops, having a volume ranging from 480 l to 2000 l and comprising a formulation according to the present invention with the preferences as given below.

It is especially advantageous if the FIBC has an "inliner", which protects its content from light, oxygen and water, as well as if it is a Type C FIBC. Openings at the top for loading and at the bottom for unloading are convenient. Therefore, such openings are preferably present. Such openings will be tightly closed during storage and transport.

### Formulation

The formulation is described in more detail below. The essential ingredients and their amounts, as well as the ingredients being absent in the formulations are further disclosed.

### Essential ingredients

### Fat-soluble vitamin

The term "fat-soluble vitamins" comprises for the purpose of the present invention vitamins A and/or D, the corresponding derivatives such as e.g. esters, especially C₁-C₂₀ alkyl esters, as well as any mixture thereof.

"Vitamin D" means either Vitamin D₃ (cholecalciferol) or Vitamin D₂ (ergocalciferol) or both.

"Vitamin D derivative" means any derivative of Vitamin D as for example 25-hydroxy vitamin D₃ (so-called "HyD"), 1,25-dihydroxy vitamin D₃ or 24,25-dihydroxy vitamin D₃.

Especially preferred examples of fat-soluble vitamins are vitamin A, vitamin A acetate, vitamin A propionate, vitamin A butanoate, vitamin A palmitate, vitamin D₃ and 25-hydroxy-vitamin D, as well as any mixture thereof. More preferred are vitamin A acetate, vitamin D₃ and any mixture thereof. Most preferred the fat-soluble vitamin is vitamin A acetate or a mixture of vitamin A acetate and vitamin D3, preferably in a weight ratio of vitamin A acetate to vitamin D3 ranging from 1:1 to 100:1, more preferably ranging from 10:1 to 85:1.

The amount of the vitamin A or its derivative thereof is chosen in such a way so that its final amount in the formulation is preferably in the range of from 25 to 58 weight-%, more preferably its final amount is in the range of from 28 to 55 weight-%, even more preferably its final amount is in the range of from 30 to 50 weight-% and from 32 to 48 weight-%, most preferably its final amount is in the range of from 33 to 46 weight-%, based on the total weight of a) to g). By disclosing these ranges any combination of any lower value with any other value to a range is also disclosed, i.e. 28-58 weight-%, 30-48 weight-%, 25-46 weight-% etc.

If a mixture of Vitamin A acetate and vitamin D3 is used, the amount of vitamin A acetate is as given above for the vitamin A derivative and the amount of vitamin D3 is chosen in such a way so that its final amount in the formulation is preferably in the range of from 0.01 to 10 weight-%, more preferably its final amount is in the range of from 0.05 to 5 weight-%, even more preferably its final amount is in the range of from 0.1 to 3.5 weight-%, most preferably its final amount is in the range of from 0.3 to 2.5 weight-%, based on the total weight of a) to g). By disclosing these ranges any combination of any lower value with any higher value to a range is also disclosed, i.e. 0.01-5 weight-%, 0.05-10 weight-%, 0.1-5 weight-% etc.

When vitamin D, preferably vitamin D₃, is present in the formulation of the present invention advantageously also an oil is present.

### Oil

The oils can be from any origin. They can be natural, modified or synthetic. If the oils are natural they can be plant or animal oils. Thus, the term "oil" encompasses any vegetable oil or fat like corn oil, sunflower oil, soybean oil, safflower oil, rapeseed oil, peanut oil, palm oil, palm kernel oil, cotton seed oil, olive oil, coconut oil, canola oil, sesame oil, hazelnut oil, almond oil, cashew oil, macadamia oil, mongongo nut oil, pracaxi oil, pecan oil, pine nut oil, pistachio oil, sacha Inchi (Plukenetia volubilis) oil, walnut oil, as well as middle chain triglycerides ("MCT") and any mixture thereof. Preferably corn oil, peanut oil, safflower oil or sunflower oil are used.

The weight ratio of vitamin D to the oil is preferably ranging from 1:1 to 1:10, more preferably from 1:2 to 1:5.

### Gelatin

Suitable gelatins are poultry gelatin, porcine gelatin, bovine gelatin and any mixture thereof, as well as fish gelatin and any mixture with the other gelatins. The gelatin is generally classified according to its Bloom value.

There are two types of gelatins: Type A gelatin is obtained from acid processing of collagen. Type B gelatin is obtained from alkaline processing of collagen.

Bloom is a test to measure the strength of a gel of gelatin. The test determines the weight (in grams) needed by a probe (normally with a diameter of 0.5 inch) to deflect the surface of the gel 4 mm without breaking it. The result is expressed in bloom (grades). In general gelatins with a Bloom ranging from 0 to 300 may be used.

Low bloom, medium bloom and high bloom gelatin are gelatins having a strength of less than about 120 Bloom (low Bloom), between about 120 and up to 200 Bloom (medium Bloom) or a strength of more than about 200 Bloom (high Bloom).

Low bloom gelatin, preferably gelatin with Bloom ranging from 0 to <120, more preferably gelatin with 60-110 Bloom, even more preferably gelatin with 70-90 Bloom, most preferably gelatin with 80 Bloom, can be used as well as medium bloom gelatin,-preferably-gelatin with 120-160 Bloom, more preferably gelatin with 140 Bloom, and high Bloom gelatin, preferably gelatin with 200-300 Bloom, more preferably gelatin with 200-270 Bloom, even more preferably gelatin with 200-250 Bloom, most preferably gelatin with 200 Bloom.

The amount of gelatin in the formulations of the present invention is chosen in such a way so that its final amount in the formulation is preferably at least 46 weight-%, more preferably at least 48 weight-%, even more preferably at least 49 weight-%, most preferably at least 50 weight-%, based on the total weight of a) to g). The maximum amount of gelatin in the formulation may preferably be 70 weight-%, more preferably 65 weight-%, even more preferably 62 weight-%, 60 weight-% and 58 weight-%, and most preferably 55 weight-%, based on the total weight of a) to g).

By disclosing these lower and higher values any combination of any lower value with any higher value to a range is also disclosed, i.e. 46-65 weight-%, 51-55 weight-%, 50-70 weight-% etc.

### Reducing sugar

Examples of suitable reducing sugars are aldohexoses and ketohexoses with a hydroxy group in α-position.

Reducing disaccharides such as e.g. lactose and maltose and reducing oligosaccharides may also be used, as well as aldopentoses and ketopentoses with a hydroxy group in α-position.

Preferred examples of reducing sugars are glucose, fructose, galactose and any mixture thereof. A suitable mixture of fructose and glucose is e.g. invert sugar. A further suitable reducing sugar mixture is high-fructose corn syrup (HFCS), also known as glucose-fructose, isoglucose and glucose-fructose syrup, a sweetener made from corn starch. "HFCS 42" and "HFCS 55" refer to 42% and 55% fructose composition respectively, the rest being glucose and water.

Hydrolysed starch products with a DE > 20, so called "glucose syrups" or "dried glucose syrups" - depending on their water content, may also be used as reducing sugars. Glucose syrup 4280, i.e. a glucose syrup with a DE ranging from 42-80 is especially suitable.

"Dextrose" is a synonym for "glucose". The term "dextrose equivalent" (DE) denotes the degree of hydrolysis and is a measure of the amount of reducing sugar calculated as D-glucose based on dry weight; the scale is based on native starch having a DE close to 0 and glucose having a DE of 100.

"Glucose syrups" or "dried glucose syrups" may be used in form of powders, microgranulates or granulates. Glucose syrups consist in general of a mixture of glucose, maltose and oligo- and polysaccharides with varying amounts of these ingredients.

When glucose syrup is used its amount is calculated on basis of the dried glucose syrup.

More preferred examples are glucose, fructose and any mixture thereof. Even more preferred a single reducing sugar is used, especially either fructose or glucose or glucose syrup with a DE ≥ 50.

Most preferred is fructose.

The amount of the reducing sugar in the formulations of the present invention is chosen in such a way so that its final amount in the formulation is preferably in the range of from 0 to 4.5 weight-%, more preferably its final amount is in the range of from 0.2 to 4.1 weight-%, most preferably its final amount is in the range of from 0.3 to 1.2 weight-%, based on the total weight of a) to g). By disclosing these ranges any combination of any lower value with any higher value to a range is also disclosed, i.e. 0-1.2 weight-%, 0.3-4.5 weight-%, etc.

### Polyhydric alcohol

Examples of suitable polyhydric alcohols are glycerol, monoesters of glycerol with C₁₋₅ monocarboxylic acids, monoethers of glycerol, diglycerol, triglycerol, polyglycerol, propylene glycol, dipropylene glycol, 1,3-butylene glycol, ethylene glycol, polyethylene glycol, sorbitol, xylitol, maltitol, erythritol, mannitol, etc.

These polyhydric alcohols can be used alone or in a combination of two or more. Preferable examples of polyhydric alcohol include glycerol, sorbitol, xylitol, maltitol, erythritol, and mannitol, whereby glycerol is especially preferred.

The amount of the polyhydric alcohol in the formulations of the present invention is chosen in such a way so that its final amount in the formulation is preferably in the range of from 0 to 5.0 weight-%, more preferably its final amount is in the range of from 0.1 to 4.1 weight-%, even more preferably its final amount is in the range of from 0.5 to 3.0 weight-%, most preferably its final amount is in the range of from 0.8 to 2.2 weight-%, based on the total weight of a) to g). By disclosing these ranges any combination of any lower value with any higher value to a range is also disclosed, i.e. 0.1-2.2 weight-%, 0.8-5.0 weight-%, etc.

### Antioxidant

The antioxidant may be a water-soluble antioxidant or a fat-soluble antioxidant or any mixture thereof. Thus, mixtures of water-soluble antioxidants, mixtures of fat-soluble antioxidants and mixtures of one or more water-soluble antioxidants and one or more fat-soluble antioxidants are also included in the term "antioxidant". Preferred are fat-soluble antioxidants as well as mixtures thereof and mixtures of water- and fat-soluble antioxidants. Inorganic antioxidants may also be present.

Examples of inorganic antioxidants are NaBH₄, Na₂SO₃ and/or Na₂S₂O₃.

Examples of fat-soluble antioxidants are ascorbyl palmitate, polyphenols, flavones being substituted with one or more hydroxy groups, isoflavones being substituted with one or more hydroxy groups, tocotrienols and analogues thereof, tocopherols and analogues thereof, phenols with bulky alkyl groups such as e.g. **b**utylated **h**ydroxy**a**nisoles ("BHA"), **b**utylated **h**ydroxy**t**oluenes ("BHT"), *tert*butylhydroquinone, thymol (= 5-Methyl-2-(propan-2-yl)phenol), eugenol (= 2-Methoxy-4-(prop-2-en-1-yl)phenol) or any mixture thereof.

Analogues of tocopherols and tocotrienols are especially compounds with a shorter side chain in position 2 compared to tocopherols and tocotrienols.

Examples of flavones substituted with one or more hydroxy groups are: 6-hydroxyflavone, 5,7-dihydroxyflavone (= chrysin), 4',5,7-trihydroxyflavone (= apigenin), 3',4',5,7-tetrahydroxyflavone (luteolin) and 4',5,6,7,8-pentamethoxy-flavone (tangeritin).

Examples of isoflavones substituted with one or more hydroxy groups and optionally methoxy groups are daidzein (= 4',7-dihydroxyisoflavone), genistein (= 4',5,7-trihydroxyisoflavone), prunetin (= 4',5-dihydroxy-7-methoxyisoflavone), biochanin A (= 5,7-dihydroxy-4'-methoxy-soflavone), orobol (= 3',4',5,7-tetrahydroxyisoflavone), santal (= 3',4',5-trihydroxy-7-methoxy-isoflavone) and pratensein (= 3',5,7-trihydroxy-4'-methoxyisoflavone).

The BHA is preferably a mixture of 2-tert-butyl-4-hydroxy-anisole and 3-tert-butyl-4-hydroxy-anisole. The BHT is preferably 2,6-di-*tert*-butyl-p-cresol (IUPAC name = 2,6-di-tert-butyl-4-methylphenol).

The use of BHA is restricted; it is e.g. not allowed in cat food any more. Thus, it is not a preferred antioxidant in the formulation of the present invention.

Examples of water-soluble antioxidants are ascorbic acid and its salts such as e.g. sodium ascorbate, citric acid and its salts such as e.g. sodium citrate, as well as any mixture thereof.

Preferred examples of mixtures of water- and fat-soluble antioxidants are tocopherol and sodium ascorbate, tocopherol and ascorbic acid, whereby the tocopherol may be alpha-, beta-, gamma- or delta-tocopherol, preferably whereby the tocopherol is alpha- or delta-tocopherol, more preferably whereby the tocopherol is alpha-tocopherol, most preferably whereby the tocopherol is DL-alpha-tocopherol.

### Tocopherols, tocotrienols and analogues thereof

Examples of suitable tocopherols and analogues thereof are e.g. compounds of formula (II) 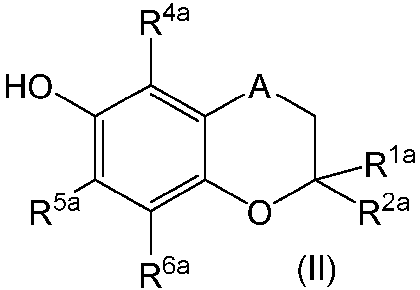 wherein R^{1a} and R^{2a} are independently from each other H or C₁₋₁₁-alkyl or (CH₂)ₙ-OH with n being an integer from 1 to 4, or R^{1a} and R^{2a} represent together a keto group, A is CHR^{3a} or C(=O), and wherein R^{3a}, R^{4a} and R^{6a} are independently from each other H or C₁₋₄-alkyl, and wherein R^{5a} is H or OH or C₁₋₄-alkyl or C₁₋₄-alkoxy, as disclosed in WO 2019/185894.

Further suitable tocopherols are compounds of formula (II), wherein one of the two substituents R^{1a} and R^{2a} is C₁₂₋₂₁-alkyl and the other of the two substituents R^{1a} and R^{2a} is either hydrogen or C₁₋₅-alkyl or (CH₂)ₙ-OH with n being an integer from 1 to 5, and wherein A is CH(R^{3a}), and wherein R^{3a}, R^{4a} and R^{6a} are independently from each other H or C₁₋₄-alkyl, and wherein R^{5a} is H or OH or C₁₋₄-alkyl or C₁₋₄-alkoxy, as disclosed in WO 2019/185938.

Compounds of formula (II), wherein A is CH₂, R^{1a} is C₁₋₅-alkyl, R^{2a} is either H or C₁₋₂-alkyl, R^{5a} is either H or C₁₋₄-alkoxy or C₁₋₄-alkyl, and R^{4a} and R^{6a} are independently from each other either H or C₁₋₄-alkyl, with the preferences as disclosed in WO 2019/185900 are also suitable antioxidants in the formulations of the present invention.

Preferred examples of the antioxidants of formula (II) as disclosed in WO 2019/185894 are the following compounds of formula (1)-(11) with "Me" being methyl:

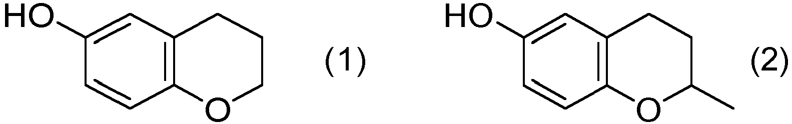

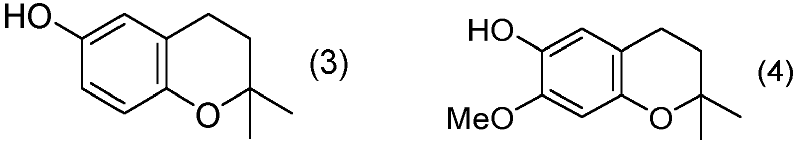

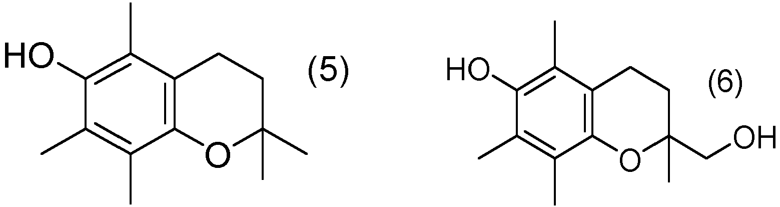

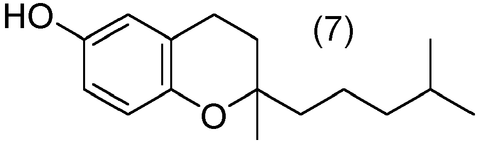

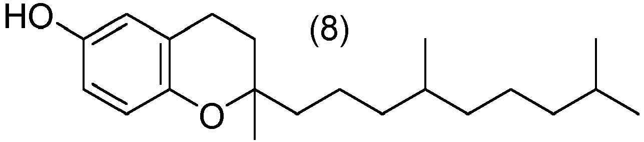

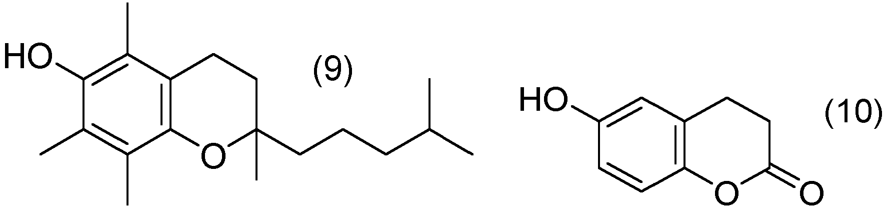

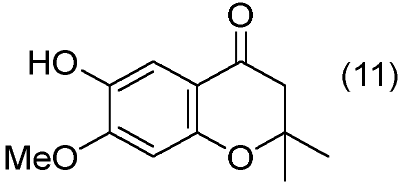

Further examples of suitable antioxidants that can be used in the formulations of the present invention are compounds of formula (III) and (IV), 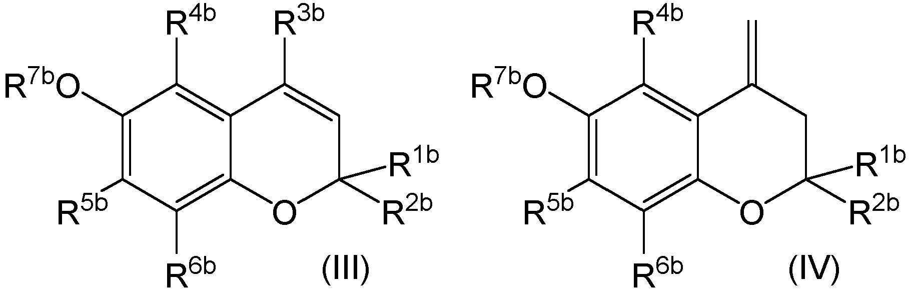 wherein R^{1b} and R^{2b} are independently from each other H or C₁₋₁₁-alkyl or (CH₂)ₙ-OH with n being an integer from 1 to 6 or R^{1b} and R^{2b} together represent a keto group, and wherein R^{3b}, R^{4b}, R^{5b}, and R^{6b} are independently from each other H or C₁₋₆-alkyl or C₁₋₆-alkoxy, and R^{7b} is H or C₁₋₆-alkyl, as disclosed in WO 2019/185898. "alkyl" and "alkoxy" hereby encompass linear alkyl and branched alkyl, and linear alkoxy and branched alkoxy, respectively.

Preferred examples of compounds of formula (III) and (IV) are the following compounds (12)-(19):

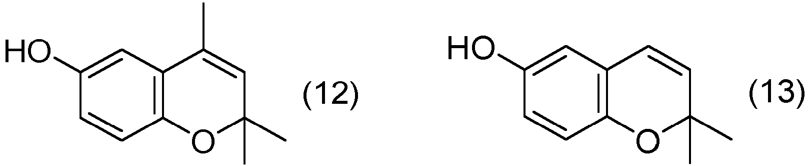

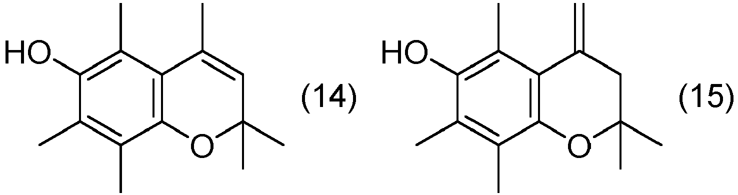

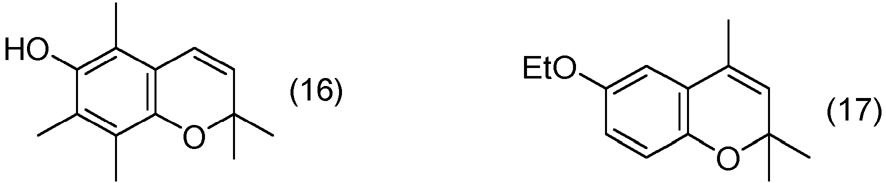

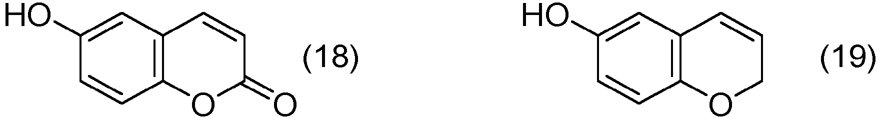

Further suitable antioxidants are compounds of formula (V), whereby R¹, R² and R³ are independently from each other H or linear C₁₋₆-alkyl or branched C₃₋₈-alkyl, whereby preferably R¹ is H or methyl or ethyl or *n*-propyl or *iso*-propyl or *tert*-butyl and R² and R³ are independently from each other H or methyl or ethyl, with the further preferences as disclosed in WO 2019/185940.

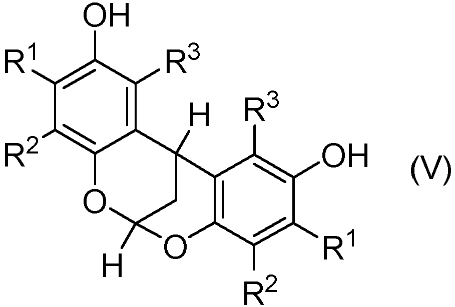

Also, the compounds of formula (VI) with n being 1 or 2, R^{1b} and R^{3b} being independently from each other H or C₁₋₅-alkyl, and R^{2b} being either H or C₁₋₅-alkyl or C₁₋₅-alkyloxy, preferably with the proviso at least one of R^{1b}, R^{2b} and R^{3b} being H, as disclosed in WO 2019/185904 can be used as antioxidants in the formulations of the present invention.

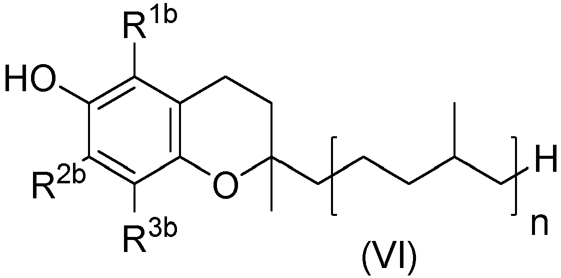

Hereby the following compounds of formulae (VI-1) and (VI-2) are especially preferred:

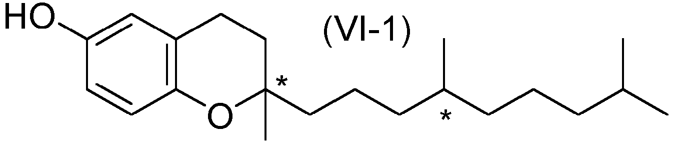

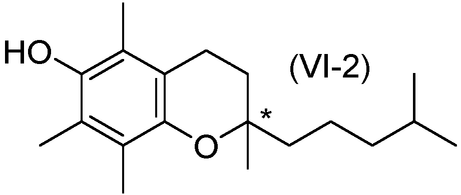

The asterisks * mark each a chiral/stereogenic center, i.e. all possible isomers having any configuration at said centers are encompassed by the term "compound of formula (VI-1)" and "compound of formula (VI-2)", respectively.

Also, suitable antioxidants are compounds of the following formulae (VII) and (VIII) with R^{1c}, R^{2c} and R^{3c} being independently from each other H or C₁₋₄-alkyl as published in WO 2019/185942 and WO 2019/185888, respectively. Preferred examples thereof are tocotrienols and tocopherols of the formulae (20) to (27) as shown below.

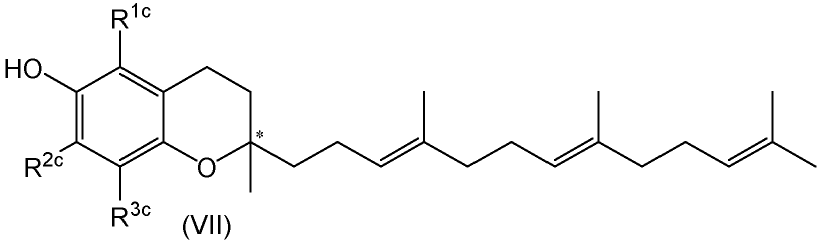

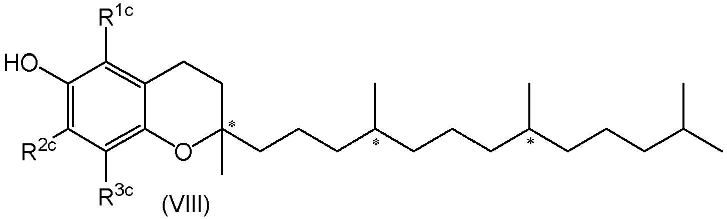

The asterisks * mark each a chiral/stereogenic center. The term "compound of formula (VII)/(VIII)" encompasses all possible isomers having any configuration at said centers.

Especially preferred examples of the compound of formula (VII) are the following compounds of formulae (20) (= alpha-tocotrienol), (21) (= beta-tocotrienol), (22) (= gamma-tocotrienol) and (23) (= delta-tocotrienol), whereby all possible diastereomers and enantiomers are included.

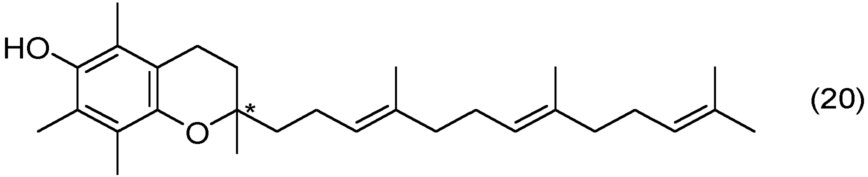

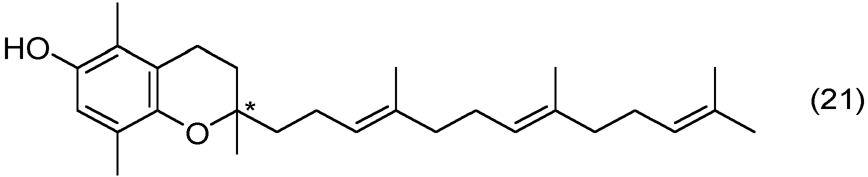

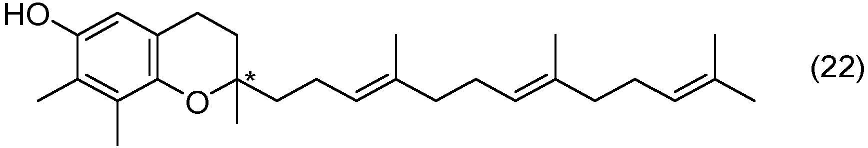

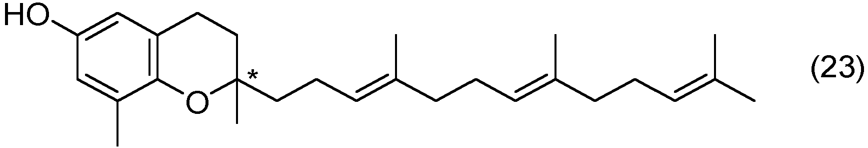

Especially preferred examples of the compound of formula (VIII) are the following compounds of formulae (20) (= alpha-tocopherol), (21) (= beta-tocopherol), (22) (= gamma-tocopherol) and (23) (= delta-tocopherol), whereby all possible diastereomers and enantiomers are included.

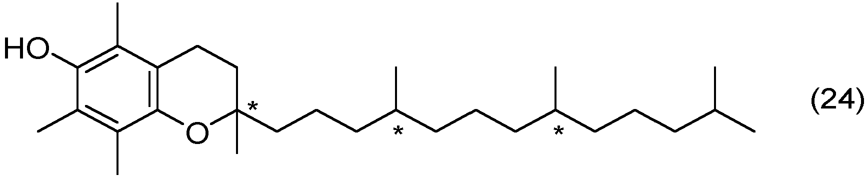

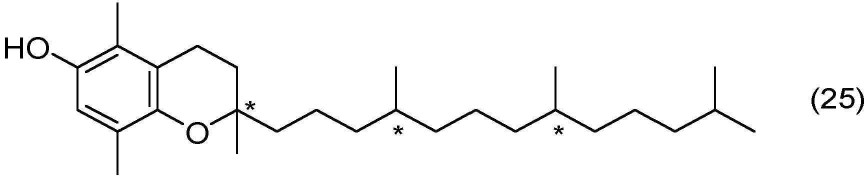

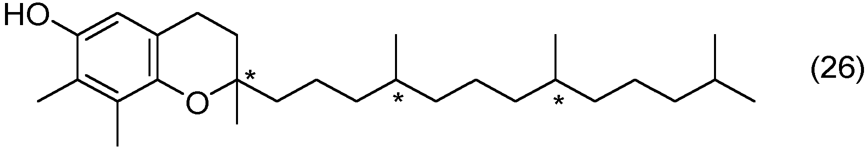

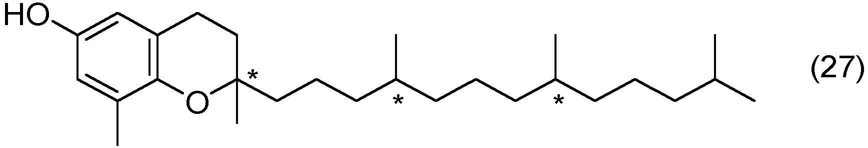

The asterisks * mark each a chiral/stereogenic center. The term "compound of formula (20)/(21)/(22)/(23)/(24)/(25)/(26)/(27)" encompasses all possible isomers having any configuration at said centers.

### Polyphenols

Examples of suitable polyphenols are 2,4,5-trihydroxybutyrophenone, epigallo-catechin gallate ("EGCG"), epigallo-catechin, gallo-catechin, hydroxytyrosol, resveratrol, carnosol, 2-(3,4-dihydroxyphenyl)acetic acid and C₁₋₆ alkyl esters thereof, and any mixture thereof.

Further suitable polyphenols are derivatives, preferably esters and (earth) alkali metal salts, of hydroxybenzoic acids such as e.g. gallic acid (= 3,4,5-trihydroxybenzoic acid) and syringic acid (= 4-hydroxy-3,5-dimethoxy-benzoic acid). Examples of preferred esters are C₁₋₂₀ alkyl esters of gallic acid such as e.g. propyl gallate, octyl gallate or dodecyl gallate, and C₁₋₂₀ alkyl esters of syringic acid.

Also, derivatives, preferably esters and (earth) alkali metal salts, of cinnamic acid and hydroxycinnamic acids such as e.g. ferulic acid (= 3-(4-hydroxy-3-methoxyphenol)prop-2-enoic acid), caffeic acid (= 3,4-Dihydroxycinnamic acid), dihydrocaffeic acid (= 3-(3,4-dihydroxyphenyl) propanoic acid), chlorogenic acid (= the ester of caffeic acid and (-)-quinic acid), o-, m-, p-coumaric acid (= 2-/3-/4-hydroxycinnamic acid), rosmarinic acid (= a caffeic acid ester of 3-(3,4-dihydroxyphenyl)lactic acid), or sinap(in)ic acid (= 3,5-dimethoxy-4-hydroxycinnamic acid) may be used as antioxidants in the present invention.

Examples of derivatives of cinnamic acid are Z-ethoxyethyl p-methoxycinnamate, ethylhexyl p-methoxycinnamate, 2-ethylhexyl 4-methoxycinnamate, methyl diisopropylcinnamate, isoamyl 4-methoxycinnamate, and diethanolamin 4-methoxycinnamate.

The most preferred antioxidants used in the formulation of the present invention are a butylated hydroxytoluene (= "BHT") such as 2,6-di-tert-butyl-p-cresol (IUPAC name = 2,6-di-tert-butyl-4-methylphenol) as well as a mixture of DL-alpha-tocopherol and sodium ascorbate, a mixture of DL-alpha-tocopherol and ascorbic acid and a mixture of DL-alpha-tocopherol and ascorbyl palmitate.

The weight ratio of DL-alpha-tocopherol to sodium ascorbate and of DL-alpha-tocopherol to ascorbic acid is preferably ranging from 5:1 to 1:5, more preferably from 3:1 to 1:3, even more preferably from 2:1 to 1:2, most preferably from 1.1:1 to 1:1.1.

Surprisingly the total amount of the antioxidant(s) in the formulations of the present invention is lower than in gelatin-based formulations already being on the market.

Preferably the total amount of the antioxidant(s) is chosen in such a way so that its/their final amount in the formulation is preferably ≤ 6.8 weight-%, more preferably its/their final amount is in the range of from 0.1 to 6.6 weight-% and from 0.2 to 5.5 weight-%, even more preferably its/their final amount is in the range of from 0.3 to 5.0 weight-% and from 0.4 to 4.8 weight-%, most preferably its/their final amount is in the range of from 0.5 to 4.6 weight-% and from 0.5 to 2.5 weight-%, based on the total weight of a) to g). By disclosing these ranges any combination of any lower value with any higher value to a range is also disclosed, i.e. at least 0.5 and at most 6.8 weight-%, 0.3 to 2.5 weight-%; etc.

In the following **Table I** the preferred amounts for specific antioxidants in addition to the ranges above are given:

**Table I: All amounts are based on the total weight of a) to g).**

| **Antioxidant** | **Preferred amount, based on the total weight of the formulation** | **More preferred amount, based on the total weight of the formulation** | **Most preferred amount, based on the total weight of the formulation** |
|---|---|---|---|
| 2,6-di-tert-butyl-p-cresol | 3.0-6.8 weight-% | 4.0-6.7 weight-% | 5.0-6.6 weight-% |
| DL-alpha-tocopherol | 0.05-5.5 weight-% | 0.1-5.0 weight-% | 0.15-2.5 weight-% |
| DL-alpha-tocopherol and ascorbic acid | 0.1-5.0 weight-% | 0.5-4.6 weight-% | 1-4.2 weight-% |
| DL-alpha-tocopherol and sodium ascorbate | 0.1-6.7 weight-%, 0.1-5.0 weight-% | 0.2-3.0 weight-%, 0.5-2.5 weight-% | 1.0-2.2 weight-% |

Also, within a row any value may be combined with any value of another column.

It is assumed that the amount of other tocopherols or analogues thereof, as well as of tocotrienols and analogues thereof is in a similar range as for DL-alpha-tocopherol. It is further assumed that the amount of other polyphenols, especially of other phenols, is in a similar range as for 2,6-di-*tert*-butyl-p-cresol.

### Anti-caking agent

Suitable organic anti-caking agents are corn starch, as well as starches from other botanical sources such as e.g. as waxy corn, wheat, tapioca, pea and potato, as well as derivatives thereof such as pre-gelatinised starch, starch ethers (e.g. carboxymethyl starch), starch esters (e.g. starch monophosphate, alkenylsuccinated starch, especially octenyl-succinated starch), cross-linked starch and oxidized starch and any mixture thereof. Other suitable organic anti-caking agents are talc, cellulose, microcrystalline cellulose, cellulose derivatives or fibres, ferric ammonium citrate, sodium salts of fatty acids such as e.g. sodium stearate, potassium salts of fatty acids such as e.g. potassium stearate, calcium salts of fatty acids such as e.g. calcium stearate, magnesium salts of fatty acids such as e.g. magnesium stearate, aluminum salts of fatty acids such as e.g. aluminum stearate, ammonium salts of fatty acids such as e.g. ammonium stearate, and any mixture of any of them.

Other suitable anti-caking agents are inorganic anti-caking agents such as e.g. silicic acid H₂ₙ₊₂SiₙO₃ₙ₊₁ and alkaline/earth alkali metal salts thereof, precipitated silicic acid, silica (= silicon dioxide), modified silica, hydrophobically modified silica, precipitated silica, magnesium oxide, dicalcium diphosphate, tricalcium phosphate, magnesium phosphate, sodium carbonate, potassium carbonate, calcium carbonate, magnesium carbonate, calcium oxide, magnesium oxide, potassium silicate, calcium silicate, magnesium silicate, magnesium trisilicate, aluminum silicate, sodium aluminum silicate, potassium aluminum silicate, calcium aluminum silicate, zeolithe (aluminosilicates), disodium sulfate or mixtures thereof. Further suitable inorganic anti-caking agents are bentonite and kaolin.

Also, mixtures of organic and inorganic anti-caking agents may be used.

The preferred anti-caking agents used in the formulations of the present invention are anti-caking agents with a particle size D(v,50%) from 100 nm to 10 µm, preferably from 100 nm to 9 µm, more preferably from 150 nm to 5 µm, measured as dry dispersion with a Malvern MasterSizer 3000 (laser diffraction).

The particle size of the anti-caking agent can be determined with a laser diffraction system e.g. Malvern MasterSizer 3000, either as dry dispersion or as wet dispersion in oil or Volasil (a mixture of volatile and cyclic silicones such as octamethylcyclotetrasiloxane and decamethylcyclopentasiloxane). The particle size can also be determined with electron microscopy.

The more preferred anti-caking agents are silicic acid H₂ₙ₊₂SiₙO₃ₙ₊₁, silica, microcrystalline cellulose, as well as any mixture thereof.

The most preferred anti-caking agents are hydrophilic precipitated silicic acid H₂ₙ₊₂SiₙO₃ₙ₊₁, hydrophilic precipitated silica and any mixture thereof.

The amount of the anti-caking agent is chosen in such a way so that its final amount in the formulation is preferably ranging from 1-10 weight-%, more preferably ranging from 3 to 9 weight-%, most preferably ranging from 4.5 to 7.5 weight-%, based on the total weight of a) to g). By disclosing these ranges any combination of any lower value with any higher value to a range is also disclosed, i.e. 1-7.5 weight-%, 4.5-10 weight-% etc.

### Not present ingredients

In contrast to the formulations disclosed in EP 494 417 A2 the matrix of the formulations of the present invention does **not** comprise any of the following salts: water-soluble salts of carboxylic acids, sodium carbonate, potassium carbonate, calcium sulfate, and calcium phosphate. Examples of such water-soluble salts of carboxylic acids not being present in the matrix of the formulations of the present invention are: aluminum subacetate, sodium tartrate, sodium glutarate, sodium acetate, calcium acetate, sodium propionate, calcium propionate and sodium benzoate. Some of these salts may, however, be used as anti-caking agents during drying of the formulation, especially during the powder-catch process step. Preferably none of these salts is used in the manufacture of the formulations of the present invention; thus, the formulations of the present invention do not comprise any of these salts; i.e. no water-soluble salts of carboxylic acids, no sodium carbonate, no potassium carbonate, no calcium sulfate, and no calcium phosphate.

In EP-A 494 417 these salts are used for the cross-linking of the gelatin with a reducing sugar at temperatures ranging from 55°C to 85°C for 2-24 hours which was believed to be necessary for stable formulations. Some embodiments of the present invention showed that cross-linking with a reducing sugar is not necessary at all (see examples 3-7 and 9) to obtain stable formulations.

The process of the present invention has the advantage that no organic solvent except water is used, so that the formulation according to the present invention is substantially free of organic solvents. "substantially free" means that the amount thereof is ≤ 5 weight-%, preferably ≤ 3 weight-%, more preferably ≤ 1 weight-%, even more preferably ≤ 0.5 weight-%, most preferably ≤ 0.1 weight-%.

The formulation of the present invention does preferably not comprise any non-reducing sugars such as e.g. sucrose instead of the reducing sugars. Mixtures of non-reducing and reducing sugars may, however, be used.

Furthermore, no cross-linking agents such as e.g. acetaldehyde, glutaraldehyde or glyoxal are present in the formulations of the present invention.

Ethoxyquin, also known as "EMQ" (IUPAC name: 6-Ethoxy-2,2,4-trimethyl-1,2-dihydroquinoline), is also not present in the formulations of the present invention; whereas according to EP-A 494 417 the presence of ethoxyquin seemed to be necessary to obtain stable formulations.

In a preferred embodiment of the present invention the formulation does not comprise ethoxyquin and it does not comprise a water-soluble salt of carboxylic acids and it does not comprise sodium carbonate and it does not comprise potassium carbonate and it does not comprise calcium sulfate and it does not comprise calcium phosphate.

Furthermore, no lignosulfonate is present in the formulations of the present invention. Non-limiting examples of lignosulfonates that are not present in the formulations of the present invention are: sodium lignosulfonate, ammonium lignosulfonate, magnesium lignosulfonate, calcium lignosulfonate; as well as any of their mixtures. Suppliers of lignosulfonates are: Borregaard Industries Limited, Norway; Burgo Group, Rayonier Advanced Materials, Wuhan Xinyingda Chemicals, Shenyang Xingzhenghe Chemical, Abelin Polymers, GREENAGROCHEM, Harbin Fecino Chemical, Karjala Pulp, Nippon Paper Industries, Pacific Dust Control, Sappi, The Dallas Group of America, Venki Chem and Xinyi Feihuang Chemical.

In a further preferred embodiment of the present invention the formulation does not comprise any of the compounds mentioned in this chapter "not present ingredients".

### Characteristics of the formulations of the present invention

The formulations of the present invention show preferably a bulk density ranging from 0.55-0.67 g/cm³, more preferably a bulk density ranging from 0.56-0.66 g/cm³, most preferably a bulk density ranging from 0.58-0.64 g/cm³.

Furthermore, the formulations of the present invention show preferably a tap density ranging from 0.58-0.71 g/cm³, more preferably a tap density ranging from 0.60-0.69 g/cm³, most preferably a tap density ranging from 0.62-0.67 g/cm³.

The bulk density and the tap density are measured as follows:
A 250 ml glass cylinder is filled with the sample. The volume and the weight are measured. Bulk density is the weight divided by the volume.

For measuring the tap density the sample is tapped with a 2000 Taps with a Stampfvolumeter JEL STAV II (J. Engelsmann AG). The tapped volume is measured. Tap density is the weight divided by the tapped volume.

Preferably the formulations of the present invention show besides the bulk density and/or tap density above, also the particle sizes and/or particle size distribution as cited below, which results in a very good homogeneity when mixed into feed.

### Preferred embodiments of the formulations of the present invention

**Table II** and **Table III** show preferred formulations of the present invention and their composition. Instead of Vitamin A acetate another Vitamin A C₁₋₂₀ alkyl ester, preferably another Vitamin A C₂₋₁₆ alkyl ester, may also be used.

The formulations according to **Table II** comprise vitamin A acetate and optionally vitamin D3, gelatin, a phenol with bulky alkyl groups as antioxidant, an anti-caking agent and optionally residual moisture, optionally a reducing sugar and optionally a polyhydric alcohol. When vitamin D3 is present, an oil in the amounts as given above is preferably also present.

The formulations according to **Table III** comprise vitamin A acetate and optionally vitamin D3, gelatin, a mixture of antioxidants, an anti-caking agent and optionally residual moisture, optionally a reducing sugar and optionally a polyhydric alcohol, When vitamin D3 is present, an oil in the amounts as given above is preferably also present.

**Table II: All amounts a) to g) are given in weight-% and are based on the total weight of a) to g). The amounts of a) to g) always sum up to 100 weight-%. The amount of moisture is given in weight-% and based on the total weight of the formulation.**

| **Ingredients** | **Preferred amount** | **More preferred amount** | **Most preferred amount** |
|---|---|---|---|
| **vitamin A acetate** | 25-55 | 30-50 | 35-48 |
| **vitamin D3** | 0.05-5 | 0.1-3.0 | 0.5-2.5 |
| **Gelatin with the preferences as given above** | 46-60 | 47-56 | 50-55 |
| **A reducing sugar with the preferences as given above** | 0.1-4.5 | 0.2-4.1 | 0.3-1.2 |
| **A polyhydric alcohol with the preferences as given above** | 0.1-5.0 | 0.5-3.0 | 0.8-2.2 |
| **a phenol with bulky alkyl groups, preferably BHT (antioxidant)** | 3.0-6.8 | 4.0-6.7 | 5.0-6.6 |
| **Anti-caking agent with the preferences as given above, preferably silicic acid or silica** | 2.0-9.0 | 3.0-8.0 | 3.5-7.0 |
| **Residual moisture** | 0.5-6.5 | 1.0-5.5 | 1.0-4.0 |

**Table III: All amounts a) to g) are given in weight-% and are based on the total weight of a) to g). The amounts of a) to g) always sum up to 100 weight-%. The amount of moisture is given in weight-% and based on the total weight of the formulation.**

| **Ingredients** | **Preferred amount** | **More preferred amount** | **Most preferred amount** |
|---|---|---|---|
| **vitamin A acetate** | 25-55 | 30-50 | 35-48 |
| **vitamin D3** | 0.05-5 | 0.1-3.0 | 0.5-2.5 |
| **Gelatin with the preferences as given above** | 48-57 | 49-56 | 50-55 |
| **A reducing sugar with the preferences as given above** | 0.1-4.5 | 0.2-4.1 | 0.3-1.2 |
| **A polyhydric alcohol with the preferences as given above** | 0.1-5.0 | 0.5-3.0 | 0.8-2.2 |
| **a tocopherol or analogue thereof, preferably DL-α-tocopherol (antioxidant)** | 0.1-5.5 | 0.5-5.2 | 0.7-5.0 |
| **A mixture of a tocopherol or analogue thereof, preferably DL-α-tocopherol (antioxidant), and a water-soluble antioxidant, preferably sodium ascorbate or ascorbic acid or any mixture thereof** | 0.1-4.6 | 0.3-4.2 | 0.5-2.1 |
| **Anti-caking agent with the preferences as given above, preferably silicic acid or silica** | 2-9 | 4-8 | 6.5-7.5 |
| **Residual moisture** | 0.5-6.5 | 1.0-5.5 | 1.0-5.0 |

Especially preferred formulations of the present invention are the following formulations A to F, whereby the amounts of the single ingredients are given in weight-% and are based on the total weight of all ingredients cited in the corresponding tables below. The amounts of all ingredients cited in the corresponding tables below always sum up to 100 weight-%. The amount of moisture is in the range of 1-4 weight-% and based on the total weight of the corresponding formulation. The formulations preferably only contain the ingredients cited in the corresponding tables and no further ingredients. Thus, the formulations preferably consist only of the ingredients as cited in the corresponding tables.

### Formulation A

| **Ingredient** | **Amount** | **Preferred amount** | **More preferred amount** | **Most preferred amount** |
|---|---|---|---|---|
| Vitamin A acetate [weight-%] | 30-45 | 35-40 | 36-39 | 37.5 |
| Gelatin [weight-%] with the preferences as cited above | 45-52 | 46-50 | 47-49 | 47.5-48.5 |
| Reducing sugar [weight-%] with the preferences as cited above | 3.0-5.0 | 3.5-4.5 | 3.7-4.2 | 4.0 |
| Polyhydric alcohol with the preferences as cited above, preferably glycerol [weight-%] | 1-3 | 1.5-2.5 | 1.8-2.2 | 2.0 |
| Fat-soluble antioxidant with the preferences as cited above, preferably DL-alpha-tocopherol [weight-%] | 0.1-2 | 0.5-1.5 | 0.8-1.2 | 1.0 |
| water-soluble antioxidant with the preferences as cited above, preferably sodium ascorbate or ascorbic acid, more preferably sodium ascorbate [weight-%] | 0-2 | 0-1.5 | 0 or 0.8-1.2 | 0 or 1.0 |
| Anti-caking agent with the preferences as cited above, preferably silica [weight-%] | 5-9 | 6-8 | 6.5-7.5 | 7.0 |

Any combination of the amount of one ingredient with the preferred amount of another ingredient and/or the more or most preferred amount of a further ingredient is also encompassed within the scope of this preferred Formulation A.

If Vitamin D3 is additionally present in the Formulation A, its amount is in the range of 2-3 weight-%, based on the total weight of the ingredients cited in the corresponding table above. Furthermore, preferably also an oil is then present with the preferences as given above. In this case the amount of gelatin is reduced accordingly.

### Formulation B

| **Ingredient** | **Amount** | **Preferred amount** | **More preferred amount** | **Most preferred amount** |
|---|---|---|---|---|
| Vitamin A acetate [weight-%] | 30-45 | 35-40 | 36-39 | 37.5 |
| Gelatin [weight-%] with the preferences as cited above | 48-53 | 49-52 | 49.5-51.5 | 50.5 |
| fat-soluble antioxidant with the preferences as cited above, preferably DL-alpha-tocopherol [weight-%] | 3-7 | 4-6 | 4.5-5.5 | 5.0 |
| Anti-caking agent with the preferences as cited above, preferably silica [weight-%] | 5-9 | 6-8 | 6.5-7.5 | 7.0 |

Any combination of the amount of one ingredient with the preferred amount of another ingredient and/or the more or most preferred amount of a further ingredient is also encompassed within the scope of this preferred Formulation B.

If Vitamin D3 is additionally present in the Formulation B, its amount is in the range of 2-3 weight-%, based on the total weight of the ingredients cited in the corresponding table above. Furthermore, preferably also an oil is then present with the preferences as given above. In this case the amount of gelatin is reduced accordingly.

### Formulation C

| **Ingredient** | **Amount** | **Preferred amount** | **More preferred amount** | **Most preferred amount** |
|---|---|---|---|---|
| Vitamin A acetate [weight-%] | 30-45 | 35-40 | 36-39 | 37.5 |
| Gelatin [weight-%] with the preferences as cited above | 50-58 | 52-56 | 53.0-54.5 | 53.5-54.0 |
| fat-soluble antioxidant with the preferences as cited above, preferably DL-alpha-tocopherol [weight-%] | 0.1-2 | 0.3-1.5 | 0.4-1.1 | 0.5 or 1.0 |
| Water-soluble antioxidant with the preferences as cited above, preferably sodium ascorbate or ascorbic acid [weight-%] | 0.1-2 | 0.5-1.5 | 0.8-1.2 | 1.0 |
| Anti-caking agent with the preferences as cited above, preferably silica [weight-%] | 5-9 | 6-8 | 6.5-7.5 | 7.0 |

Any combination of the amount of one ingredient with the preferred amount of another ingredient and/or the more or most preferred amount of a further ingredient is also encompassed within the scope of this preferred Formulation C.

If Vitamin D3 is additionally present in the Formulation C, its amount is in the range of 2-3 weight-%, based on the total weight of the ingredients cited in the corresponding table above. Furthermore, preferably also an oil is then present with the preferences as given above. In this case the amount of gelatin is reduced accordingly.

### Formulation D

| **Ingredient** | **Amount** | **Preferred amount** | **More preferred amount** | **Most preferred amount** |
|---|---|---|---|---|
| Vitamin A acetate [weight-%] | 33-39 | 34-38 | 35-37 | 36.2 |
| Gelatin [weight-%] with the preferences as cited above | 48-52.8 | 49.4-51.4 | 49.9-50.9 | 50.4 |
| Reducing sugar [weight-%] with the preferences as cited above | 0.1-2 | 0.5-1.5 | 0.8-1.2 | 1.0 |
| Polyhydric alcohol with the preferences as cited above, preferably glycerol [weight-%] | 0.1-2 | 0.5-1.5 | 0.8-1.2 | 1.0 |
| 2,6-Di-*tert*-butyl-p-cresol [weight-%] | 3.0-6.8 | 4.0-6.7 | 5.0-6.6 | 6.4 |
| Anti-caking agent with the preferences as cited above, preferably silica [weight-%] | 3-7 | 4-6 | 4.5-5.5 | 5.0 |

Any combination of the amount of one ingredient with the preferred amount of another ingredient and/or the more or most preferred amount of a further ingredient is also encompassed within the scope of this preferred Formulation D.

If Vitamin D3 is additionally present in the Formulation D, its amount is in the range of 2-3 weight-%, based on the total weight of the ingredients cited in the corresponding table above. Furthermore, preferably also an oil is then present with the preferences as given above. In this case the amount of gelatin is reduced accordingly.

### Formulation E

| **Ingredient** | **Amount** | **Preferred amount** | **More preferred amount** | **Most preferred amount** |
|---|---|---|---|---|
| Vitamin A acetate [weight-%] | 33-39 | 34-38 | 35-37 | 36.2 |
| Vitamin D3 [weight-%] | 1-3.5 | 1.8-2.8 | 2.0-2.5 | 2.2 |
| Gelatin [weight-%] with the preferences as cited above | 48.8-51.8 | 49.3-51.3 | 49.8-50.8 | 50.3 |
| 2,6-Di-*tert*-butyl-p-cresol [weight-%] | 3.0-6.8 | 4.0-6.7 | 5.0-6.6 | 6.4 |
| Anti-caking agent with the preferences as cited above, preferably silica [weight-%] | 3-7 | 4-6 | 4.5-5.5 | 5.0 |

Any combination of the amount of one ingredient with the preferred amount of another ingredient and/or the more or most preferred amount of a further ingredient is also encompassed within the scope of this preferred Formulation E.

If Vitamin D3 and/or an oil is/are not present in the Formulation E, the amount of gelatin is increased accordingly.

### Formulation F

| **Ingredient** | **Amount** | **Preferred amount** | **More preferred amount** | **Most preferred amount** |
|---|---|---|---|---|
| Vitamin A acetate [weight-%] | 30-45 | 35-40 | 36-39 | 37.5 |
| Gelatin [weight-%] with the preferences as cited above | 52-57 | 53-56 | 53.5-55.5 | 54.5 |
| Fat-soluble antioxidant with the preferences as cited above, preferably DL-alpha-tocopherol [weight-%] | 0.1-2 | 0.5-1.5 | 0.8-1.2 | 1.0 |
| Anti-caking agent with the preferences as cited above, preferably silica [weight-%] | 5-9 | 6-8 | 6.5-7.5 | 7.0 |

Any combination of the amount of one ingredient with the preferred amount of another ingredient and/or the more or most preferred amount of a further ingredient is also encompassed within the scope of this preferred Formulation F.

If Vitamin D3 is additionally present in the Formulation F, its amount is in the range of 2-3 weight-%, based on the total weight of the ingredients cited in the corresponding table above. Furthermore, preferably also an oil is then present with the preferences as given above. In this case the amount of gelatin is reduced accordingly.

Formulation F with the most preferred amounts of the ingredients as given above has shown self-heating temperatures ≥ 120°C and ΔT < 60 K, independently on whether gelatin Bloom 80 or gelatin Bloom 140 or gelatin Bloom 200 has been used in its manufacture. It showed also a good process stability (i. e. loss of Vitamin A acetate ≤ 21%) in a typical broiler diet based on maize/wheat/soybean meal when pelleted at 90°C for 20 seconds to produce the feed comprising such formulation F.

### Particle size and measurement

To determine the particle size of the solid formulation of the present invention a sieve test can be performed with filters of 850 µm pores (mesh 20), 425 µm pores (mesh 40) and 150 µm pores (mesh 100).

Furthermore, the particle size of the solid formulation may also be determined by laser diffraction analytic whereby the dry dispersion of the sample is measured with a Malvern Mastersizer 2000 or 3000 and Fraunhofer calculation.

The particle sizes of the formulation, when determined with laser diffraction with a Malvern Mastersizer 3000 and Fraunhofer calculation, are as follows:
D(v,10%) = 130-290 µm, preferably 160-260 µm;
D(v, 50%) = 180-350 µm, preferably 200-330 µm;
D(v, 90%) = 260-470 µm, preferably 280-455 µm.

Synonyms of "D(v,10%)" are "d(v,10%)", "d(v,10)", "d(v,0.1)", "D(v,10)" and "D(v,0.1)". This applies in an analogous manner for "D(v,50%)" and "D(v,90%)".

### Process for the manufacture of the formulation

The present invention is also directed to a process for the manufacture of a formulation with the preferences as cited above comprising the following steps:
A) Dissolving gelatin b) and, if present, a water-soluble antioxidant c) in water to obtain a matrix;
B) Heating the fat-soluble vitamin a), the fat-soluble antioxidant c) and, if present, the oil to obtain an active phase;
C) Emulsifying the active phase obtained in step B) into the matrix obtained in step A) to obtain a dispersion;
D) Spray-drying the dispersion obtained in step C) in presence of an anti-caking agent to obtain the formulation;
whereby a reducing sugar and/or a polyhydric alcohol may optionally be added, preferably either to the matrix in step A) or in step C).

Preferably the process for the manufacture of the formulation according to the present invention does neither comprise a crosslinking step performed by exposure to radiation nor does it comprise a crosslinking step performed by using an enzyme.

The formulation of the present invention is preferably manufactured according to a process comprising the following steps:
A) Dissolving gelatin b), the reducing sugar f), the polyhydric alcohol g) and, if present, a water-soluble antioxidant c) in water to obtain a matrix;
B) Heating the fat-soluble vitamin a), the fat-soluble antioxidant c) and, if present, an oil e) to obtain an active phase;
C) Emulsifying the active phase obtained in step B) into the matrix obtained in step A) to obtain a dispersion;
D) Spray-drying the dispersion obtained in step C) in presence of an anti-caking agent e) to obtain the formulation.

Alternatively, one or both of the two ingredients, the reducing sugar f) and the polyhydric alcohol g), may not be added to the water or matrix formed by the gelatin in step A), but to the mixture in step C) before or during emulsification.

The single steps are disclosed in more detail below. Further details, that may also be generalized, are given in the examples.

### Step A)

The amounts of the gelatin b), the reducing sugar f), the polyhydric alcohol g) and if present, the water-soluble antioxidant c) are chosen so that the final amounts of these compounds in the solid formulation after having performed steps A) to D) and A) to E), respectively, is as described above.

Step A) is preferably performed at a temperature ranging from 40 to 80°C, more preferably ranging from 50 to 75°C, most preferably ranging from 55 to 70°C.

### Step B)

The amounts of the fat-soluble vitamin a), the fat-soluble antioxidant c) and, if present, the oil e) are chosen so that the final amounts of these compounds in the solid formulation after having performed steps A) to D) and A) to E), respectively, is as described above.

Step B) is preferably performed at a temperature to bring the components a) and c) into a liquid state.

When vitamin D, preferably vitamin D₃, is present in the formulation of the present invention, the vitamin D is preferably added to the other fat-soluble vitamin and the fat-soluble antioxidant as oily suspension, whereby the weight ratio of vitamin D to the oil is preferably ranging from 1:1 to 1:10, more preferably from 1:2 to 1:5.

If only vitamin D, preferably vitamin D₃, is present in the formulation of the present invention, the vitamin D is preferably added to the fat-soluble antioxidant as oily suspension, whereby the weight ratio of vitamin D to the oil is preferably ranging from 1:1 to 1:10, more preferably from 1:2 to 1:5

### Step C)

Preferably this step is performed at a mixing temperature in the range of from 40 to 75°C, more preferably at a mixing temperature in the range of 50 to 70°C, even more preferably at a mixing temperature in the range of 55°C to 65°C to obtain a dispersion.

The homogenization can be achieved by using a rotor-stator device or a highpressure homogenizer or both. Other devices known to the person skilled in the art may also be used.

### Step D)

The dividing and drying of the mixture of the oil-in-water preparation to produce the solid formulation according to the present invention can be done in any conventional way, such as spray cooling, modified spray cooling, spray drying, spray-drying in combination with fluidized bed granulation, modified spray drying or sheet drying and crushing, see e.g. WO 91/06292 A1.

Preferably the conversion to the solid formulation is achieved by a powder-catch technique, whereby the sprayed dispersion droplets are caught by the anti-caking agent (so-called "catch media"), and dried.

### Step E)

It is advantageous to treat the powder obtained after having performed step D) thermally. Hereby preferably temperatures of up to 125°C are applied, more preferably the thermal treating is carried out at a temperature ranging from 80-120°C, even more preferably the thermal treating is carried out at a temperature ranging from 100 to 120°C, most preferably the thermal treating is carried out at a temperature ranging from 110 to 120°C. Hereby the amount of moisture in the formulation is decreased.

### Further embodiments of the present invention

### Use

The present invention is also directed to the use of the formulation according to the present invention with the preferences as given above as additive to feed or premixes.

### Feed additive, premix and feed according to the present invention

The present invention is also directed to a feed additive, a premix and a feed comprising the formulation according to the present invention with the preferences as given above. Feed (or 'feedingstuff') means any substance or product, including additives, whether processed, partially processed or unprocessed, intended to be used for oral feeding to animals.

Feed in the context of the present invention is especially feed for broilers including starter, grower, finisher; broiler breeders including starter, grower (pullets), layers and male breeders, for layers and other poultry such as e.g. hens and duck layers, layers breeders, ducks and geese, partridges, quails and pheasants, ostrich and emu, for turkeys including starter, grower, finisher; for turkey breeders including starter, grower, layers and male breeders, for ruminants including calves, milk replacer, heifers, beef cattle, breeding bulls, sheep and goats; for horses, especially foals, leisure horses, race horses, mares and stallions, for rabbits, for mick and foxes, for swine including fattening pigs: pre-starter, starter, grower, finisher; breeders: replacement gilts, sows, boars, and feed for companion animals, especially for dogs and cats.

The amount of the formulation and the fat-soluble vitamin respectively follows the regulatory guidelines in the regions depending on the specific animal species and its age.

In the Supplementation Guidelines the amount of the vitamins A and D₃ is given in International Units ("I.U.").

To ensure that the active content in the feed is communicated in a systematic way, "I.U." is used as a universal unit for fat soluble vitamins because there are different forms of the vitamins with varying amounts of fat-soluble vitamins.

The formulation according to the present invention is usually added to feed in form of a premix, i.e. a mixture with other micro-nutrients such as other vitamins or their formulations and minerals. The premix inclusion in feed is < 1 weight-% for many species.

The amount of the formulation according to the present invention needed to be included into the feed is calculated based on the active content of the feed and the targeted dosage of the fat-soluble vitamin in the final feed considering said inclusion level.

The conversion factors of the fat-soluble vitamins are as follows:
1 I.U. Vitamin A corresponds to 0.344 µg of Vitamin A acetate;
1 I.U. Vitamin D₃ corresponds to 0.025 µg of Vitamin D₃.

The following **Table IV** shows the amounts of the fat-soluble vitamins added per kg of air-dry feed. The exact amount is depending on several factors such as phase/age of the animal, animal species and legal local limits.

**Table IV**

| **Animal** | **Vitamin A [I.U.]** | **Vitamin D₃ [I.U.]** | **HyD [mg]** |
|---|---|---|---|
| **Broilers** | 10000-15000 | 3000-5000 | - |
| **Turkeys** | 6000-15000 | 3000-5000 | 0.092 |
| **Layers & other poultry** | 8000-16000 | 3000-5000 | 0.069 |
| **Swine** | 5000-20000 | 1000-2000 | 0.05 |
| **Ruminants** | 20000-150000 | 400-40000 | - |
| **Horses** | 25000-80000 | 3500-8500 | - |
| **Rabbits** | 8000-12000 | 800-1200 | - |
| **Mink & Foxes** | 10000-15000 | 1500-2000 | - |
| **Dogs** | 7100-23000 | 640-1100 | - |
| **Cats** | 9000-25000 | 750-1300 | - |
| **Fish & Shrimps** | 4000-12000 | 1500-6500 | - |

Below are given non-limiting examples of feed to which the formulations of the present invention may be added.

### Feed for poultry

The feed for poultry differs from region to region. In the following **Tables V and VI** typical examples for diets in Europe and Latin America are given. These diets include cereals such as wheat, rye, maize/corn, minerals such as NaCl, vegetable oils such as soya oil, amino acids and proteins.

Thus, the present invention is also directed to feed for poultry comprising the formulation according to the present invention; preferably to feed for poultry comprising the formulation according to the present invention and cereals, minerals, vegetable oils, amino acids and proteins.

**Table V: European diet**

| **Ingredients (%)** | **Starter Period (day 0-21)** | **Grower Period (day 22-36)** |
|---|---|---|
| Wheat | 20.00 | 22.50 |
| Rye | 12.00 | 12.00 |
| Soybean meal | 34.00 | 28.50 |
| Maize | 27.00 | 28.50 |
| Vegetable Oil | 3.10 | 4.20 |
| NaCl | 0.10 | 0.10 |
| DL Methionine | 0.24 | 0.24 |
| L-Lysine | 0.15 | 0.15 |
| Limestone | 0.85 | 0.85 |
| Dicalcium Phosphate | 1.50 | 1.90 |
| Vitamin & Mineral mix | 1.00 | 1.00 |
| Coccidiostat (Avatec) | 0.06 | 0.06 |
| TiO₂ | - | 0.10 |
| calculated Provision | | |
| apparent metabolizable energy, MJ/kg | 12.5 | 12.90 |
| apparent metabolizable energy, kcal/kg | 2986 | 3082 |
| crude Protein, % | 21.2 | 19.1 |
| Methionine + Cysteine, % | 0.89 | 0.83 |
| Lysine, % | 1.23 | 1.09 |
| Calcium, % | 0.83 | 0.91 |
| total phosphorus, % | 0.68 | 0.73 |
| available phosphorus, % | 0.35 | 0.40 |

**Table VI: Latin American diet**

| **Ingredients (%)** | **Starter** | **Grower** |
|---|---|---|
| Corn | 53.0 | 57.1 |
| Soybean meal | 38.5 | 34.2 |
| Calcium | 0.70 | 0.70 |
| Phosphorus | 2.40 | 2.00 |
| NaHCO₃ | 0.23 | 0.24 |
| NaCl | 0.20 | 0.20 |
| Methionine | 0.30 | 0.10 |
| Lysine | 0.21 | 0.00 |
| Soya Oil | 3.50 | 4.50 |
| Premix | 1.00 | 1.00 |

| Calculated provision (%) | | |
|---|---|---|
| Crude protein | 22.4 | 20.4 |
| apparent metabolizable energy, (MJ/kg) | 12.7 | 13.2 |
| apparent metabolizable energy, (kcal/kg) | 3034 | 3154 |
| Total phosphorus | 0.86 | 0.76 |
| Calcium | 1.00 | 0.85 |
| Available phosphorus | 0.44 | 0.38 |
| d-Lysine | 1.25 | 0.98 |
| d-Methionine + Cysteine | 0.91 | 0.68 |
| d-Threonine | 0.77 | 0.71 |
| Na | 0.18 | 0.18 |
| Cl | 0.20 | 0.19 |

### Pet food

Pet foods are formulated to meet nutrient specifications using combinations of multiple ingredients to meet the targeted nutrient specification.

The nutrient specifications for a complete and balanced dog or cat food will meet or exceed the guidelines provided by AAFCO (American Association of Feed Control Officials). The ingredient composition of pet-food can include any legal feed ingredient so number of combinations are not quite infinite but close. Some examples of ingredient used in dog and cat foods can be found in **Table VII** below:

**Table VII:**

| **Ingredient Class/Ingredient** | **Use rates** |
|---|---|
| **1 ANIMAL MEALS** | **10-35%** |
| Chicken | |
| Turkey | |
| Duck | |
| Poultry Br-Product | |
| Lamb | |
| Venison | |
| Beef | |
| Pork | |
| Meat & Bone | |
| Fish | |

| **2 FRESH MEATS** | **3-20%** |
|---|---|
| Chicken | |
| Turkey | |
| Duck | |
| Lamb | |
| Venison | |
| Beef | |
| Pork | |
| Fish | |

| **3 VEGETABLE PROTEINS** | **8-20%** |
|---|---|
| Soybean Meal | |
| Corn Gluten Meal | |
| Pea Protein | |
| Potato Protein | |
| Soy Protein Conc/Isolates | |

| **4 GRAINS** | **0-70%** |
|---|---|
| Corn/Maize | |
| Wheat | |
| Brown Rice/Brewers Rice | |
| Oatmeal/Oat Groats | |
| Barley | |
| Millet | |
| Milo/Sorghum | |
| Rye | |
| Corn Gluten Feed | |
| Wheat Middlings | |

| **5 FIBER SOURCES** | **2-8%** |
|---|---|
| Beet Pulp | |
| Corn Bran | |
| Wheat Bran | |
| Cellulose | |
| Tomato Ponace | |
| Potato Fiber | |
| Pea Fiber | |

| **6 FATS & OILS** | **1-15%** |
|---|---|
| Animal Fat | |
| Poultry Fat | |
| Chicken Fat | |
| Beef Tallow | |
| Sunflower Oil | |
| Canola Oil | |

| **7 MICRONUTRIENTS** | **0.10-1%** |
|---|---|
| Vitamins | |
| Minerals | |
| Others (e.g. Fructooligosaccharides (FOS) used as a pre-biotic) | |

| **8 PALATANTS (FLAVORS)** | **0-5%** |
|---|---|
| **9 Other non-basic ingredients** | |
| Dried Egg Product | 1-15% |
| Fish Oil | 0.5-2% |
| Fish Meal | 1-4% |
| Flaxseed | 1-4% |
| Dried Peas | 5-30% |
| Dried Chickpeas | 5-30% |
| Dried Lentils | 5-10% |
| Dried Potatoes | 5-20% |
| Dried Sweet Potatoes | 5-20% |
| Tapioca Starch | 5-15% |
| Potato Starch | 5-15% |
| Pea Starch | 5-15% |

Thus, the present invention is also directed to pet food comprising the formulation according to the present invention; preferably to pet food comprising the formulation according to the present invention and animal meals and/or fresh meats, vegetable proteins, grains, fiber sources, fats and/or oils, micronutrients, palatants and optionally other non-basic ingredients.

### Feed for Swine

Reference is made here to the NATIONAL SWINE NUTRITION GUIDE, 2010, whereby two non-limiting examples are given below.

**Table VIII: Corn and Soybean Meal Diet**

| **DIET** | **PERCENTAGE, %** |
|---|---|
| Corn, yellow | 70.99 |
| Soybean meal, solv. extr., CF<4%, CP<48% | 25.19 |
| Corn oil | 1.00 |
| Calcium carbonate | 0.95 |
| Monocalcium phosphate | 0.78 |
| Salt (NaCl) | 0.37 |
| L-Lysine HCl | 0.17 |
| DL-Methionine | 0.04 |
| L-Threonine | 0.02 |
| Vitamin and trace mineral premix | 0.50 |
| Total | 100% |

**Table IX: High fiber ingredient diet**

| **DIET** | **PERCENTAGE, %** |
|---|---|
| Corn, yellow | 37.48 |
| Corn DDGS, <4% oil | 30.00 |
| Wheat middlings | 19.00 |
| Soybean meal, solv. extr., CF<4%, CP<48% | 7.11 |
| Corn oil | 3.52 |
| Calcium carbonate | 1.28 |
| Salt (NaCl) | 0.39 |
| L-Lysine HCl | 0.57 |
| DL-Methionine | 0.03 |
| L-Tryptophan | 0.04 |
| L-Threonine | 0.10 |
| Vitamin and trace mineral premix | 0.50 |
| Total | 100% |

Thus, the present invention is also directed to feed for swine comprising the formulation according to the present invention; preferably to feed for swine comprising the formulation according to the present invention and corn, soybean meal, minerals, vegetable oils, amino acids, further vitamins and trace mineral premixes.

The invention is now further illustrated in the following non-limiting examples.

### Examples

The following examples 1-9 illustrate the manufacture of the formulations of the present invention at different scale.

In the tables all amounts are given in weight-% and are based on the total weight of the formulation excluding the residual moisture. The amounts of all ingredients except the residual moisture always sum up to 100 weight-%.

Example 10 shows the stability of a formulation according to the present invention in a premix. Example 11 shows the stability of a formulation according to the present invention under pelleting conditions.

### Example 1: Formulation of the present invention with low amount of antioxidant

The used ingredients and their amounts in the final formulation are given in **Table 1** below. As gelatin, bovine gelatin bloom 140-200 is used. As a fat-soluble antioxidant dl- α-tocopherol is used. As a water-soluble antioxidant sodium ascorbate is used. As reducing sugar glucose syrup 4280 (i.e. DE = 42-80, i.e. a high glucose content), as commercially available from Roquette(France), is used.

The gelatin, the reducing sugar, the glycerol and the water-soluble antioxidant are dissolved in deionized water to obtain the so-called "matrix". The crystalline vitamin A and the fat-soluble antioxidant are heated at 65°C under stirring in a water bath until the complete melting of the crystals ("active phase").

Subsequently, the active phase is pre-emulsified into the matrix at 5500 rpm by using a rotor stator (Esco, Singapore). Afterwards, the emulsion is further emulsified at 6000 rmp for 30 min.

Subsequently, the emulsion is sprayed into silica. The solid formulation remains in the catch medium silica for 45 to 60 minutes. The thus obtained dried beadlets are sieved and the fraction with a particle size in the range of 160 to 630 µm is used.

### Comparison example 1

Comparison example 1 is carried according to the process of example 1. The used ingredients and their amounts in the final formulation are given in **Table 1** below. The formulation according to the comparison examples has higher amounts of sugar, glycerol and antioxidant compared to the formulation of example 1. Despite these higher amounts of sugar, glycerol and antioxidant the self-heating temperature of comparison example 1 is lower than the one of example 1.

### Examples 2-7

Example 2 illustrates the manufacture of a formulation according to the present invention that comprises a reduced amount of reducing sugar and a reduced amount of polyhydric alcohol as compared to commercially available formulations (see comparison example 1). Examples 3-7 illustrate the manufacture of formulations according to the present invention that neither comprise reducing sugars nor polyhydric alcohols.

### General procedure I

The matrix components, i.e. reducing sugar (if present), glycerol (if present), gelatin and optionally the water-soluble antioxidant are dissolved in water at approximately 65°C to obtain the "matrix".

Vitamin A acetate, Vitamin D3 (if present) and the fat-soluble antioxidant are heated at approximately 65°C under stirring until complete melting of vitamin A acetate ("active phase"). They are then emulsified into the matrix.

Hereby, the amounts of the ingredients are chosen in such a way that their concentrations in the final formulation are as disclosed in **Table 1** and **Table 2,** respectively.

After thorough mixing, the resulting dispersion is sprayed into a spray tower in the presence of an anti-caking agent to form droplets of the desired size. The solidified droplets are then dried by an drying air of various temperatures (5-75° C). The dried powder is separated from the majority of the anti-caking agent and sieved through 150 µm and 600 µm filters.

The powder is further treated at a temperature of up to 120°C in a mixer or in a fluid bed, whereby it is made partially water-insoluble.

The particle size of the dried and thermically-treated powder is determined with laser diffraction analytic with a Malvern Mastersizer 3000. The sample is hereby dry dispersed. By applying the Fraunhofer theory the particle size distribution of the sample is calculated.

Furthermore, the bulk and the tap density are measured according to procedures known to the person skilled in the art.

The self-heating temperature is determined according to the procedure disclosed below.

### Examples 8-9 (see Table 3)

These examples are prepared at large scale in an analogous way according to General Procedure I described above. The composition of the formulations and their parameters are given in **Table 3** below. **Example 8** illustrates the manufacture of a formulation according to the present invention that comprises a reduced amount of reducing sugar and a reduced amount of polyhydric alcohols as compared to commercially available formulations. **Example 9** illustrates the manufacture of a formulation according to the present invention that neither comprises reducing sugars nor polyhydric alcohols.

### Determination of the self-heating temperature

The tests are performed according to Norm E-15188 and VDI 2263 Part 1.

The samples are placed in a cubic shaped wire basket (either 16 ml or 1000 ml) in the center of an oven. The temperature of the oven is measured at two spots and remains constant during the testing period (24 hours), i.e. so called "isoperibolic test".

The temperature probe for the sample is placed in the center of the sample. Then the sample temperature reaches a temperature being 2 K below the oven temperature the 24 hour testing period starts.

When the temperature difference "ΔT" between sample and oven is < 60 K, the test criteria according to UN Orange book "Manual of Tests and Criteria, 6^{th} revised edition, chapter 33.3, Division 4.2 is passed, and the sample is considered as being self-heating stable according to the present invention.

When the temperature difference "ΔT" between sample and oven is > 60 K, the sample is considered as undergoing spontaneous ignition or dangerous self-heating.

As can be seen from **Tables 1, 2 and 3** all formulations according to examples 1-9 show a self-heating temperature ≥ 120°C and a ΔT < 60 K. Thus, all formulations according to examples 1-9 can be transported in FIBCs of volumes up to 3000 l, preferably in FIBCs of volumes ranging from 450 to 3000 l.

If any of examples 1-9 is repeated, but instead of gelatin Bloom 140-200, low Bloom gelatin, such as e. g. gelatin Bloom 80, is used, the thus obtained formulations also have a self-heating temperature ≥ 120°C.

If any of examples 1-9 is repeated, but instead of gelatin Bloom 140-200, high bloom gelatin, such as e. g. gelatin Bloom 200, is used, the thus obtained formulations also have a self-heating temperature ≥ 120°C.

**Table 1**

| **Examples / Ingredients** | **Comparison Example 1** | **Example 1** | **Example 2** (moisture content: 2.2 weight-%) |
|---|---|---|---|
| **Vitamin A acetate [weight-%]** | 35.7 | 37.5 | 37.5 |
| **Gelatin [weight-%]** | 35.3 | 48.5 | 47.5 |
| **Reducing sugar [weight-%]** | 6.5 | 4.0 (glucose syrup) | 4.0 (glucose) |
| **Glycerol [weight-%]** | 4.0 | 2.0 | 2.0 |
| **2,6-Di-*tert*-butyl-p-cresol [weight-%]** | 11.5 | - | - |
| **DL-alpha-tocopherol [weight-%]** | - | 1.0 | 1.0 |
| **Sodium ascorbate [weight-%]** | - | - | 1.0 |
| **Anti-caking agent (silica) [weight-%]** | 5.0 | 7.0 | 7.0 |
| **Particle size D(v,10) [µm]** | Not determined | Not determined | 188 |
| **Particle size D(v,50) [µm]** | Not determined | Not determined | 237 |
| **Particle size D(v,50) [µm]** | Not determined | Not determined | 301 |
| **Bulk density** | Not determined | Not determined | 0.61 |
| **Tap density** | Not determined | Not determined | 0.65 |
| **No self-heating at a temperature of** | 110°C | 130°C | 130°C |
| **ΔT** | Not determined | Not determined | 38K |

**Table 2**

| **Ingredients / Examples** | **Example 3** | **Example 4** | **Example 5** | **Example 6** | **Example 7** |
|---|---|---|---|---|---|
| **Vitamin A acetate [weight-%]** | 37.5 | 37.5 | 37.5 | 37.5 | 37.5 |
| **Vitamin D3 [weight-%]** | - | 2.15 | 2.15 | - | - |
| **Gelatin [weight-%]** | 50.5 | 51.35 | 51.35 | 53.5 | 54.0 |
| **DL-alpha-tocopherol [weight-%]** | 5.0 | 1.0 | 1.0 | 1.0 | 0.5 |
| **Water-soluble antioxidant [weight-%]** | - | 1.0 (sodium ascorbate) | 1.0 (ascorbic acid) | 1.0 (sodium ascorbate) | 1.0 (sodium ascorbate) |
| **Silica (anti-caking agent) [weight-%]** | 7.0 | 7.0 | 7.0 | 7.0 | 7.0 |
| **Residual moisture [weight-%]** | 1.7 | 1.9 | 1.4 | 3.4 | 1.8 |
| **Particle size d(v,10%) [pm]** | 171 | 191 | 171 | 199 | 244 |
| **Particle size d(v,50%) [µm]** | 249 | 289 | 265 | 273 | 316 |
| **Particle size d(v,90%) [µm]** | 374 | 422 | 435 | 376 | 409 |
| **Bulk density [g/cm3]** | 0.62 | 0.58 | 0.63 | 0.64 | 0.62 |
| **Tap density [g/cm3]** | 0.65 | 0.62 | 0.66 | 0.67 | 0.66 |
| **No self-heating at a temperature of** | 125°C | 130°C | 120°C | 135°C | 140°C |
| **ΔT** | 46K | 59K | 42K | 38K | 37K |

**Table 3**

| **Examples / Ingredients** | **Example 8** | **Example 9** |
|---|---|---|
| **Vitamin A acetate [weight-%]** | 36.2 | 36.2 |
| **Vitamin D3 [weight-%]** | - | 2.2 |
| **Gelatin [weight-%]** | 50.4 | 50.3 |
| **Reducing sugar [weight-%]** | 1.0 (fructose) | - |
| **Glycerol [weight-%]** | 1.0 | - |
| **2,6-Di-t*e*rt-butyl-p-cresol [weight-%]** | 6.4 | 6.4 |
| **Anti-caking agent [weight-%]** | 5.0 (silica) | 5.0 (silica) |
| **Particle size D(v,50%) [µm]** | 278-334 (average of 4 trials: 304) | 280-294 (average of 6 trials: 287) |
| **No self-heating at a temperature of** | 4 trials: 3 x 125°C, 1 x 130° C | 6 trials: 4 x 125°C, 1 x 120°C, 1 x 130° C |
| **ΔT** | 34-42K (average of 4 trials: 38K) | 29-43K (average of 6 trials: 42K) |

### Example 10: Testing of the stability of the formulation according to example 5 in a premix

The premix "P25br" in which the stability of the formulation according to example 5 is examined has the composition as shown in **Table 7.** The amount of the formulation added per kg of premix is calculated so that a targeted Vitamin A content in the premix of 2'500'000 I.U./ kg is reached.

**Table 7: Composition of the premix**

| **Ingredient** | **Form** | **Premix P25br [g/kg]** |
|---|---|---|
| Vitamin A | Formulation according to example 5 | 2.5 |
| Vitamin D3 | ROVIMIX D3 500 | 1.0 |
| Vitamin E | ROVIMIX E 50 Ads | 8.0 |
| Vitamin B2 | ROVIMIX B2 80-SD | 1.0 |
| Vitamin B1 | ROVIMIX B1 | 0.50 |
| Vitamin C | Rovimix C-EC | 50.0 |
| Choline | Choline chloride 70% | 214.0 |
| Carotenoids | CAROPHYLL Red 10% | 10.0 |
| Minerals | Mn Oxide 62% | 60.0 |
| | Zn Oxide 78% | 12.0 |
| | Fe Sulfate monohydr., 32% | 20.0 |
| | Cu Sulphate | 6.0 |
| | Co Carbonate 5% BMP | 0.2 |
| | Sepiolite | 20.0 |
| Carrier | Wheat middlings | 87.8 |
| | Limestone | 507.5 |
| Total | | 1000 |

This premix contains choline chloride in a high amount, as well as Cu and Zn. Choline chloride and high concentrations of Cu and Zn are known to reduce the stability of vitamin A (see e.g. Pan Yang et al., Animals 2019, 9, 1154 ff.). Thus, the chosen premix "P25br" is quite aggressive. If such premix shows a retention of vitamin A > 70% after storage for one month at 30°C and 65% of relative humidity that means that the vitamin A formulation used has a good stability in premixes.

### Sample preparation and assay

The ingredients to make 3.0 kg of P25br are mixed for around 10 minutes. Then the formulation according to example 5 is added. Three samples per time point are taken and packed in plastic bags.

The vitamin A content of the samples is analyzed right after the sample preparation ("initial")and after storage for one month at 30°C and 65% of relative humidity ("1 month stability") and 2 months at 30°C and 65% of relative humidity ("2 months stability").

The average retentions and coefficient variations are calculated. They are as follows:

### Premix stability of the formulation according to example 5:

Retention vs. target calculated on measured vitamin A potency: 110.3% ± 3.3%. Average retention of Vitamin A after 1 month storage at 30°C and 65% of relative humidity vs. initial Vitamin A content: 73.4% ± 4.4%.

Average retention of Vitamin A after 3 months storage at 30°C and 65% of relative humidity vs. initial Vitamin A content: 63.9 ± 4.7%.

Thus, the formulation according to the present invention has a good stability in premixes.

### Example 11: Testing of the stability of the formulation according to example 9 in feed

The feed "WMaisF11", a wheat-corn-based broiler diet, in which the stability of the formulation according to example 9 is examined has the composition as shown in **Table 8.** The amount of the formulation added per kg of premix is calculated so that a targeted Vitamin A content in the premix of 50'000 I.U./kg is reached.

**Table 8: Composition of the feed: All amounts are given in weight-% and sum up to 100% when the formulation of example 9 is included.**

| **Feed type** | **Broiler WMaisF11** |
|---|---|
| Wheat | 20 |
| Corn | 35.52 |
| Soya 48 | 36.93 |
| Soya Oil | 2.88 |
| Di-calcium phosphate | 2.17 |
| Limestone | 0.74 |
| Salt | 0.47 |
| Methionine | 0.2 |
| Lysine | 0.08 |
| Mineral premix 5245 | 1 |
| Total | 100 |

For each batch, 24.75 kg of mash feed is added to a Forberg Type F-60 mixer. The formulation of example 9 is added as a 1% dilution with wheat middlings (premix of 250 g, including 12.5 g of test product). Subsequently, test batches are pelleted in a Bühler DFPL pellet mill, using the parameters given in here:

| *Process setting* | |
|---|---|
| Mixing time, minutes | 2 |
| Die size, mm | 3.5x 20 |
| Pelleting temperature, °C | 90 |
| Moisture final feed, % | <13 |

| *Process control* | |
|---|---|
| Conditioning temperature, °C | 90 |
| Moisture conditioned feed, % | 14.7 |
| Pellet tem perature, ° C | 90.8 |
| Moisture final dried feed, % | 11.2 |

Sampling for each batch starts after around half of the time to make a batch is elapsed. Three samples ('a, b,c) per batch are collected and packed in plastic bags.

The samples are analyzed for their vitamin A content - after mixing with the mash feed and after pelleting. To increase precision, per batch three samples are analyzed by double determination. Recoveries are calculated as average of these measures and coefficient variations are calculated based on average values.

Initial pellet vs initial mash (referred as Process stability) (calculated as Vitamin A found in initial pellet sample divided by Vitamin A content found in mash feed sample): 98%±2.0%.

Example 9 demonstrates that the formulations of the present invention are highly stable under pelleting conditions.

## Claims

1. A formulation with a self-heating temperature ≥ 120° C, determined according to the method of the description, whereby said formulation comprises
a) a fat-soluble vitamin in an amount of at least 25 weight-%, whereby the fat-soluble vitamin is vitamin A or a derivative thereof and optionally vitamin D or a derivative thereof;
b) gelatin in an amount of at least 45 weight-%;
c) at least one antioxidant in an amount of ≤ 7 weight-%;
d) an anti-caking agent;
e) optionally an oil;
f) optionally a reducing sugar;
g) optionally a polyhydric alcohol;
h) optionally residual moisture;
whereby all amounts a) to g) sum up to 100 weight-% and are based on the total weight of the a), b), c), d), e), f) and g) together, and
whereby ethoxyquin is not present in the formulation.

2. The formulation according to claim 1, whereby the formulation does not comprise the following salts: water-soluble salts of carboxylic acids, sodium carbonate, potassium carbonate, calcium sulfate, and calcium phosphate, whereby the water-soluble salts of carboxylic acids not being present in the formulation are preferably: aluminum subacetate, sodium tartrate, sodium glutarate, sodium acetate, calcium acetate, sodium propionate, calcium propionate and sodium benzoate.

3. The formulation according to claim 1 and/or 2, whereby the amount of gelatin is at least 46 weight-%, preferably at least 48 weight-%, more preferably at least 50 weight-%, most preferably at least 51 weight-%, based on the total weight of the formulation.

4. The formulation according to any one or more of the preceding claims, whereby said formulation has a bulk density ranging from 0.58 to 0.64 g/cm³.

5. The formulation according to any one or more of the preceding claims, wherein the formulation has a particle size D(v,50%) ranging from 180 to 350 µm, measured as dry dispersion with a Malvern MasterSizer 3000 (laser diffraction).

6. The formulation according to any one or more of the preceding claims, wherein said anti-caking agent d) has a particle size D(v,50%) ranging from 100 nm to 10 µm, measured as dry dispersion with a Malvern MasterSizer 3000 (laser diffraction).

7. The formulation according to any one or more of the preceding claims, wherein the fat-soluble vitamin a) is vitamin A acetate or a mixture of vitamin A acetate and vitamin D3, preferably in a weight ratio of 1:1 to 100:1, more preferably from 10:1 to 85:1

8. The formulation according to any one or more of the preceding claims, whereby said antioxidant c) is a tocopherol or a mixture of a tocopherol with a water-soluble antioxidant.

9. The formulation according to any one or more of the preceding claims, whereby the formulation comprises a reducing sugar, preferably in an amount from 0.2 to 4.5 weight-%, and a polyhydric alcohol, preferably in an amount from 0.1 to 5.0 weight-%, all amounts based on the total weight of a) to g).

10. The formulation according to claim 9, whereby the reducing sugar is glucose, fructose, galactose, high-fructose corn syrup, glucose syrup, dried glucose syrup, and any mixture thereof, and/or whereby the polyhydric alcohol is glycerol, sorbitol, xylitol, maltitol, erythritol, mannitol and any mixture thereof.

11. A process for the manufacture of a formulation according to any one or more of the claims 1-10 comprising the following steps:
A) Dissolving gelatin b) and, if present, a water-soluble antioxidant c) in water to obtain a matrix;
B) Heating the fat-soluble vitamin a), the fat-soluble antioxidant c) and, if present, the oil to obtain an active phase;
C) Emulsifying the active phase obtained in step B) into the matrix obtained in step A) to obtain a dispersion;
D) Spray-drying the dispersion obtained in step C) in presence of an anti-caking agent to obtain the formulation;
whereby a reducing sugar and/or a polyhydric alcohol may optionally be added.

12. The process according to claim 11 further comprising an additional step:
E) Thermal treating the formulation obtained in step D).

13. Use of the formulation according to any one or more of claims 1-10 as additive to feed or premixes.

14. Feed additive, premix or feed comprising the formulation according to any one or more of claims 1-10.

## Patentansprüche

1. Formulierung mit einer Selbsterhitzungstemperatur ≥ 120 °C, bestimmt nach dem Verfahren der Beschreibung, wobei die Formulierung umfasst
a) ein fettlösliches Vitamin in einer Menge von wenigstens 25 Gew.-%, wobei das fettlösliche Vitamin Vitamin A oder ein Derivat davon und gegebenenfalls Vitamin D oder ein Derivat davon ist;
b) Gelatine in einer Menge von wenigstens 45 Gew.-%;
c) wenigstens ein Antioxidationsmittel in einer Menge von ≤ 7 Gew.-%;
d) ein Antibackmittel;
e) gegebenenfalls ein Öl;
f) gegebenenfalls einen reduzierenden Zucker;
g) gegebenenfalls einen mehrwertigen Alkohol;
h) gegebenenfalls Restfeuchte;
wobei die Mengen a) bis g) eine Summe von 100 Gew.-% bilden und auf das Gesamtgewicht von a), b), c), d), e),
f) und g) zusammen bezogen sind, und
wobei kein Ethoxyquin in der Formulierung vorhanden ist.

2. Formulierung nach Anspruch 1, wobei die Formulierung die folgenden Salze nicht umfasst: wasserlösliche Salze von Carbonsäuren, Natriumcarbonat, Kaliumcarbonat, Calciumsulfat und Calciumphosphat, wobei die wasserlöslichen Salze von Carbonsäuren, die nicht in der Formulierung enthalten sind, vorzugsweise sind: Aluminiumsubacetat, Natriumtartrat, Natriumglutarat, Natriumacetat, Calciumacetat, Natriumpropionat, Calciumpropionat und Natriumbenzoat.

3. Formulierung nach Anspruch 1 und/oder 2, wobei die Menge an Gelatine wenigstens 46 Gew.-%, vorzugsweise wenigstens 48 Gew.-%, bevorzugter wenigstens 50 Gew.-%, höchst bevorzugt wenigstens 51 Gew.-%, bezogen auf das Gesamtgewicht der Formulierung, beträgt.

4. Formulierung nach einem oder mehreren der vorstehenden Ansprüche, wobei die Formulierung eine Schüttdichte in dem Bereich von 0,58 bis 0,64 g/cm³ aufweist.

5. Formulierung nach einem oder mehreren der vorstehenden Ansprüche, wobei die Formulierung eine Partikelgröße D(v,50%) in dem Bereich von 180 bis 350 µm aufweist, gemessen als trockene Dispersion mit einem Malvern Mastersizer 3000 (Laserbeugung).

6. Formulierung nach einem oder mehreren der vorstehenden Ansprüche, wobei das Antibackmittel d) eine Partikelgröße D(v,50%) in dem Bereich von 100 nm bis 10 µm aufweist, gemessen als trockene Dispersion mit einem Malvern Mastersizer 3000 (Laserbeugung).

7. Formulierung nach einem oder mehreren der vorstehenden Ansprüche, wobei das fettlösliche Vitamin a) Vitamin A-Acetat oder ein Gemisch von Vitamin A-Acetat und Vitamin D3 ist, vorzugsweise in einem Gewichtsverhältnis von 1:1 bis 100:1, bevorzugter von 10:1 bis 85:1.

8. Formulierung nach einem oder mehreren der vorstehenden Ansprüche, wobei das Antioxidationsmittel c) ein Tocopherol oder ein Gemisch eines Tocopherols mit einem wasserlöslichen Antioxidationsmittel ist.

9. Formulierung nach einem oder mehreren der vorstehenden Ansprüche, wobei die Formulierung einen reduzierenden Zucker, vorzugsweise in einer Menge von 0,2 bis 4,5 Gew.-%, und einen mehrwertigen Alkohol, vorzugsweise in einer Menge von 0,1 bis 5,0 Gew.-%, umfasst, wobei alle Mengen auf das Gesamtgewicht von a) bis g) bezogen sind.

10. Formulierung nach Anspruch 9, wobei der reduzierende Zucker Glucose, Fructose, Galactose, Maissirup mit hohem Fructosegehalt, Glucosesirup, getrockneter Glucosesirup und ein beliebiges Gemisch davon ist, und/oder wobei der mehrwertige Alkohol Glycerin, Sorbit, Xylit, Maltit, Erythrit, Mannit oder ein Gemisch davon ist.

11. Verfahren zur Herstellung einer Formulierung nach einem oder mehreren der Ansprüche 1-10, umfassend die folgenden Schritte:
A) Lösen von Gelatine b) und, falls vorhanden, eines wasserlöslichen Antioxidationsmittels c) in Wasser, um eine Matrix zu erhalten;
B) Erhitzen des fettlöslichen Vitamins a), des fettlöslichen Antioxidationsmittels c) und, falls vorhanden, des Öls, um eine aktive Phase zu erhalten;
C) Emulgieren der bei Schritt B) erhaltenen aktiven Phase in die bei Schritt A) erhaltene Matrix, um eine Dispersion zu erhalten;
D) Sprühtrocknen der bei Schritt C) erhaltenen Dispersion in Gegenwart eines Antibackmittels, um die Formulierung zu erhalten;
wobei gegebenenfalls ein reduzierender Zucker und/oder ein mehrwertiger Alkohol zugesetzt werden kann.

12. Verfahren nach Anspruch 11, ferner umfassend einen zusätzlichen Schritt:
E) Wärmebehandeln der bei Schritt D) erhaltenen Formulierung.

13. Verwendung der Formulierung nach einem oder mehreren der Ansprüche 1-10 als Zusatz zu Futtermittel oder Vormischungen.

14. Futtermittelzusatz, Vormischung oder Futtermittel, umfassend die Formulierung nach einem oder mehreren der Ansprüche 1-10.

## Revendications

1. Formulation ayant une température d'auto-échauffement ≥ 120 °C, déterminée selon le procédé de la description, ladite formulation comprenant
a) une vitamine liposoluble en une quantité d'au moins 25 % en poids, la vitamine liposoluble étant la vitamine A ou un dérivé de celle-ci et éventuellement la vitamine D ou un dérivé de celle-ci ;
b) de la gélatine en une quantité d'au moins 45 % en poids ;
c) au moins un antioxydant en une quantité ≤ 7 % en poids ;
d) un agent anti-agglomérant ;
e) éventuellement une huile ;
f) éventuellement un sucre réducteur ;
g) éventuellement un alcool polyhydrique ;
h) éventuellement de l'humilité résiduelle ;
toutes les quantités a) à g) totalisant 100 % en poids et étant basées sur le poids total de a), b), c), d), e),
f) et g) ensemble, et
de l'éthoxyquine n'étant pas présente dans la formulation.

2. Formulation selon la revendication 1, dans laquelle la formulation ne comprend pas les sels suivants : sels hydrosolubles d'acides carboxyliques, carbonate de sodium, carbonate de potassium, sulfate de calcium et phosphate de calcium, les sels solubles dans l'eau d'acides carboxyliques qui ne sont pas présents dans la formulation sont de préférence : sous-acétate d'aluminium, tartrate de sodium, glutarate de sodium, acétate de sodium, acétate de calcium, propionate de sodium, propionate de calcium et benzoate de sodium.

3. Formulation selon la revendication 1 et/ou 2, dans laquelle la quantité de gélatine est d'au moins 46 % en poids, de préférence d'au moins 48 % en poids, de manière plus préférée d'au moins 50 % en poids, de manière particulièrement préférée d'au moins 51 % en poids, par rapport au poids total de la formulation.

4. Formulation selon l'une quelconque ou plusieurs des revendications précédentes, dans laquelle ladite formulation a une masse volumique apparente allant de 0,58 à 0,64 g/cm³.

5. Formulation selon l'une quelconque ou plusieurs des revendications précédentes, dans laquelle la formulation a une taille de particule D(v,50%) allant de 180 à 350 µm, mesurée comme dispersion sèche avec un MasterSizer 3000 de Malvern (diffraction laser).

6. Formulation selon l'une quelconque ou plusieurs des revendications précédentes, dans laquelle ledit agent anti-agglomérant d) a une taille de particule D(v,50%) allant de 100 nm à 10 µm, mesurée comme dispersion sèche avec un MasterSizer 3000 de Malvern (diffraction laser).

7. Formulation selon l'une quelconque ou plusieurs des revendications précédentes, dans laquelle la vitamine liposoluble a) est l'acétate de vitamine A ou un mélange d'acétate de vitamine A et de vitamine D3, de préférence dans un rapport pondéral de 1:1 à 100:1, de manière plus préférée de 10:1 à 85:1.

8. Formulation selon l'une quelconque ou plusieurs des revendications précédentes, dans laquelle ledit antioxydant c) est un tocophérol ou un mélange d'un tocophérol avec un antioxydant soluble dans l'eau.

9. Formulation selon l'une quelconque ou plusieurs des revendications précédentes, dans laquelle la formulation comprend un sucre réducteur, de préférence en une quantité de 0,2 à 4,5 % en poids, et un alcool polyhydrique, de préférence en une quantité de 0,1 à 5,0 % en poids, toutes les quantités étant basées sur le poids total de a) à g).

10. Formulation selon la revendication 9, dans laquelle le sucre réducteur est le glucose, le fructose, le galactose, le sirop de maïs à haute teneur en fructose, le sirop de glucose, le sirop de glucose séché et tout mélange de ceux-ci, et/ou dans laquelle l'alcool polyhydrique est le glycérol, le sorbitol, le xylitol, le maltitol, l'érythritol, le mannitol et tout mélange de ceux-ci.

11. Procédé de fabrication d'une formulation selon l'une quelconque ou plusieurs des revendications 1-10 comprenant les étapes suivantes :
A) dissoudre la gélatine b) et, s'il y en a, un antioxydant soluble dans l'eau c) pour obtenir une matrice ;
B) chauffer la vitamine liposoluble a), l'antioxydant liposoluble c) et, s'il y en a, l'huile pour obtenir une phase active ;
C) émulsifier la phase active obtenue à l'étape B) dans la matrice obtenue à l'étape A) pour obtenir une dispersion ;
D) sécher par pulvérisation la dispersion obtenue à l'étape C) en présence d'un agent anti-agglomérant pour obtenir la formulation ;
un sucre réducteur et/ou un alcool polyhydrique pouvant éventuellement être ajouté(s).

12. Procédé selon la revendication 11, comprenant en outre une étape supplémentaire :
E) traitement thermique de la formulation obtenue à l'étape D).

13. Utilisation de la formulation selon l'une quelconque ou plusieurs des revendications 1-10 comme additif pour un aliment pour animaux ou des prémélanges.

14. Additif alimentaire, prémélange ou aliment pour animaux comprenant la formulation selon l'une quelconque ou plusieurs des revendications 1-10.
